# EUROPEAN PATENT SPECIFICATION

(11) **EP 2 996 721 B1**
(45) Date of publication and mention of the grant of the patent: **06.10.2021**
(21) Application number: 14798014.8
(22) Date of filing: 13.05.2014
(51) Int. Cl.: A61K 39/395, A61P 35/00

(54) **COMPOSITIONS FOR TREATMENT OF ADAM8-EXPRESSING CANCER**
ZUSAMMENSETZUNGEN ZUR BEHANDLUNG VON ADAM8-EXPRIMIERENDEM KREBS
COMPOSITIONS POUR TRAITER UN CANCER EXPRIMANT ADAM8

(30) Priority: 13.05.2013 US 201361822738 P; 01.11.2013 US 201361898830 P
(43) Date of publication of application: 23.03.2016
(73) Proprietor: Tufts University, Boston, MA 02111 (US)
(72) Inventor: SONENSHEIN, Gail, Brookline Massachusetts 02446 (US); ROMAGNOLI, Mathilde, 75014 Paris (FR); MINEVA, Nora, Brighton Massachusetts 02135 (US)
(74) Representative: Fleuchaus, Andrea
(86) International application number: PCT/US2014/037857
(87) International publication number: WO 2014/186364

(56) References cited:
- EP-A1- 2 336 363
- WO-A1-2005/090991
- WO-A1-2005/090991
- WO-A2-01/09189
- US-A1- 2003 113 764
- US-A1- 2006 275 288
- STONE A L ET AL: "STRUCTURE-FUNCTION ANALYSIS OF THE ADAM FAMILY OF DISINTEGRIN-LIKE AND METALLOPROTEINASE-CONTAINING PROTEINS (REVIEW)", JOURNAL OF PROTEIN CHEMISTRY, SPRINGER SCIENCE+BUSINESS MEDIA B.V, US, vol. 18, no. 4, 1 May 1999 (1999-05-01), pages 447-465, XP008007604, ISSN: 0277-8033, DOI: 10.1023/A:1020692710029
- DATABASE MEDLINE [Online] US NATIONAL LIBRARY OF MEDICINE (NLM), BETHESDA, MD, US; 13 December 2002 (2002-12-13), SCHLOMANN UWE ET AL: "The metalloprotease disintegrin ADAM8. Processing by autocatalysis is required for proteolytic activity and cell adhesion.", XP002764977, Database accession no. NLM12372841 & SCHLOMANN UWE ET AL: "The metalloprotease disintegrin ADAM8. Processing by autocatalysis is required for proteolytic activity and cell adhesion.", THE JOURNAL OF BIOLOGICAL CHEMISTRY 13 DEC 2002, vol. 277, no. 50, 13 December 2002 (2002-12-13), pages 48210-48219, ISSN: 0021-9258
- MARTIN ET AL.: 'The Role of ADAM 15 in Glomerular Mesangial Cell Migration' THE JOURNAL OF BIOLOGICAL CHEMISTRY vol. 277, no. 37, 28 June 2002, pages 33683 - 33689, XP002557693
- ASHER ET AL.: 'Two separate metalloproteinase activities are responsible for the shedding and processing of the NG2 proteoglycan in vitro' MOLECULAR AND CELLULAR NEUROSCIENCE vol. 29, 01 May 2005, pages 82 - 96, XP004901304
- STONE ET AL.: 'Structure-Function Analysis of the ADAM Family of Disintegrin-Like and Metalloproteinase-Containing Proteins (Review' JOURNAL OF PROTEIN CHEMISTRY vol. 18, no. 4, 01 May 1999, pages 447 - 465, XP008007604
- ROMAGNOLI MATHILDE ET AL: "ADAM8 expression in invasive breast cancer promotes tumor dissemination and metastasis.", EMBO MOLECULAR MEDICINE 02 2014, vol. 6, no. 2, 2014, pages 278-294, ISSN: 1757-4684

## Description

### Technical Field

Methods are provided for prognosing, diagnosing, and treating cancers associated with ADAM8, for reducing or preventing metastasis of established tumors, and for identifying drugs that target ADAM8.

### Government Support

This invention was made with government support under grants CA129129 and ES011624 awarded by the National Institutes of Health and grant W81XWH-10-1-1003 awarded by the U.S. Army. The government has certain rights in the invention.

### Background

Cancer metastasis results from a multistep process that selects for invasive tumor cells capable of escaping from the primary site and colonizing distant organs. Early in the tumorigenesis process, the hypoxic stress that characterizes the low tissue oxygen microenvironment of a solid tumor can stimulate tumor-induced angiogenesis, which in turn supplies nutrients and oxygen necessary to prevent tumor dormancy and support tumor growth, as well as provides routes for tumor cell dissemination (Harris et al. Nat Rev Cancer 2:38-47, 2002; Majmundar et al. Mol Cell 40:294-309, 2010). Secretion of proangiogenic mediators by tumor cells, most notably vascular endothelial growth factor A (VEGF-A), promotes endothelial cell migration and proliferation (Adams and Alitalo Nat Rev Mol Cell Biol 8:464-478, 2007). The resulting neovascularization of the surrounding stroma facilitates the spreading of tumor cells into the blood stream. The process involves adherence to and transmigration through the endothelium, in particular via processes involving integrins (Avraamides et al. Nat Rev Cancer 8:604-617, 2008; Mierke J Biophys 2008:183516, 2008). Circulating tumor cells (CTCs) can be measured in the peripheral blood even before the tumor is clinically detectable (Eyles et al. J Clin Invest 120:2030-2039, 2010). The concentration of CTCs in breast cancer patients has been associated with high risk for disease recurrence and poor outcome (Cristofanilli et al. N Engl J Med 351:781-791, 2004). Moss et al. shows a method for predicting cancer disease status using assays of urine samples with a fluorescent substrate to detect metalloproteinase (MMP) activity of a large number of MMP family members, including ADAM8 and ADAM12 (WO 2012/088105 A2 published June 28, 2012).

More than 500,000 women worldwide die yearly of metastatic breast disease despite recent advances in the treatment of cancer according to reports from the World Health Organization. Identifying new therapeutic targets to effectively block breast cancer dissemination is crucial to improve the overall survival of patients at high risk. In particular, women with triple-negative breast cancer (TNBC) whose tumors are characterized as negative for estrogen receptor, progesterone receptor, and HER2 (also known as HER2/neu) lack targeted therapy (Boyle Ann Oncol 23 Suppl 6:vi7-12, 2012).

WO0109189 discloses increased levels of ADAM8 mRNA in breast cancer tissue.

### Summary

The present invention refers in various embodiments to an ADAM8 ectodomain-binding antibody or antibody fragment inhibiting the metalloproteinase domain (MP) and the disintegrin domain (DI) for the use in the treatment of breast cancer or breast cancer associated metastasis in a subject. Furthermore, the ADAM8 ectodomain-binding antibody or antibody fragment is characterized by binding to SEQ ID No. 9.The application further describes ADAM8 cancer being selected from breast, brain, lung, prostate, skin, ovarian, colorectal, bone, renal, head and neck, esophageal, gastric, liver, bladder, cervical, uterine, thyroid, testicular, and pancreatic cancers.

In one embodiment, the breast cancer to be treated is selected from the group consisting of: estrogen receptor negative, progesterone receptor negative, HER2 negative, triple-negative inflammatory, triple-negative, basal-like, invasive, ductal, ductal carcinoma *in situ* (DCIS), lobular, HER2 positive, and mixed breast cancer. The application further includes treating the cancer or metastasis by observing a decrease in size of an established tumor or a decrease in size or frequency of metastases.

According to the application, the antibody can be obtained by immunizing with a peptide from an ADAM8 protein having an amino acid sequence selected from any of: SEQ ID NO: 4; SEQ ID NO: 5; SEQ ID NO: 6; and SEQ ID NO: 7.. Alternatively, the antibody is obtained by immunizing with a peptide including an amino acid sequence from ADAM8 protein amino acid residues 158 to 653 corresponding to the ectodomain.

The ADAM8 activity in various embodiments is selected from the group consisting of: ADAM8 metalloproteinase enzyme activity; release of VEGF-A; promotion of angiogenesis; and extravasation or intravasation of metastatic cancer cells through vascular endothelium; and activation of β1-integrin.

In various embodiments, the use further includes administering to the patient a therapeutic agent which is a therapeutically effective amount of at least one antibody or antibody fragment and a pharmaceutically acceptable carrier.

In the application the use further includes selecting the at least one antibody or antibody fragment that binds at least one extracellular domain selected from a group consisting of a metalloproteinase (MP) and a disintegrin (DI), for administration to the patient.

In certain embodiments, the antibody or antibody fragment binds to and inhibits the activity of a plurality of ectodomains, for example, the metalloproteinase domain and the disintegrin domain. According to the application, the antibody or antibody fragment is recombinantly produced.

According to the application, the antibody or antibody fragment is at least one of IgG, IgM, and IgA.

According to the application, the antibody or antibody fragment is monoclonal. Alternatively, the antibody or antibody fragment is polyclonal.

According to the application, at least one of the antibody, the antibody fragment, or a binding region of the antibody or antibody fragment is selected from the group of: a single chain antibody (scFv), a recombinant camelid heavy-chain-only antibody (VHH), a shark heavy-chain-only antibody (VNAR), a microprotein, a darpin, an anticalin, an adnectin, an aptamer, an Fv, a Fab, a Fab', and a F(ab')2.

According to the application, the antibody or antibody fragment is an immunoconjugate.

According to the application, the immunoconjugate antibody or antibody fragment is bound to at least one of: a chemotherapeutic agent, a photosensitizing agent, a cytotoxic natural product, a phytotoxin, a cytotoxic antibiotic, a bacterial toxin, a fungal toxin, a bioactive protein, a radioisotype, an enzyme, and one or more enzymes that activates an inert pre-cytotoxic agent.

According to the application, the antibody or antibody fragment includes a first antibody or antibody fragment that binds to a disintegrin domain (DI) and a second antibody or antibody fragment that binds to a metalloproteinase domain (MP).

According to the application, after administering the treatment further includes measuring a decrease of at least one symptom selected from the group consisting of: primary tumor size, number of metastatic tumors, size of metastatic tumors, formation of new metastatic lesions, angiogenesis, and concentration of circulating tumor cells.

An aspect of the invention provides a pharmaceutical composition that includes a therapeutically effective amount of at least one antibody, antibody fragment, or compound that binds specifically binds and inhibits an ADAM8 domain or a portion of at least one domain of ADAM8 selected from the group: metalloproteinase domain and a disintegrin domain. An embodiment provides the composition formulated for systemic delivery to a subject. The application further provides the route of delivery being an injection. The pharmaceutical composition is delivered to the subject that is a human. Alternatively, the subject is a high value animal.

According to the application, the pharmaceutical composition further includes at least one therapeutic agent selected from the group consisting of: a growth factor, an anti-inflammatory agent, an ADAM8 knockdown, and an inhibitor of ADAM8 synthesis. According to the application, the ADAM8 knockdown composition is at least one of the group: short interfering RNA (siRNA), short hairpin RNA (shRNA), and microRNA (miRNA) inhibitor of ADAM8 synthesis.

According to the application, the vasopressor agent of the pharmaceutical composition is at least one selected from the group consisting of: nitric oxide, calcium channel blockers, collagenase inhibitors, topical steroids, matrix metalloproteinase inhibitors, ascorbates, angiotensin II, angiotensin III, calreticulin, tetracyclines, fibronectin, collagen, thrombospondin, transforming growth factors, (TGF), keratinocyte growth factors (KGF), fibroblast growth factor (FGF), insulin-like growth factors (IGF), IGF binding proteins, epidermal growth factors (EGF), platelet derived growth factor (PDGF), neu differentiation factor, hepatocyte growth factor, vascular endothelial growth factor, heparin-binding EGF, von Willebrand Factor-C, heparin, heparin sulfates, and hyaluronic acid.

According to the application, the pharmaceutical composition further includes at least one from the group consisting of an anti-coagulant agent, anti-tumor agent, anti-viral agent, anti-bacterial agent, anti-myobacterial agent, anti-fungal agent, anti-proliferative agent, and anti-apoptotic agent.

According to the application the composition is formulated in a unit dosage.

According to the application, the antibody or antibody fragment of the pharmaceutical composition is monoclonal. Alternatively, the antibody or antibody fragment is polyclonal. In various embodiments, the antibody, antibody fragment, or compound is provided in a dosage effective to decrease at least one symptom selected from the group consisting of: growth or angiogenesis of a primary or metastatic tumor, presence of circulating tumor cells, and appearance of new metastases. According to the application the composition, the antibody or antibody fragment is recombinantly produced.

According to the application the pharmaceutical composition, the antibody, antibody fragment, or at least one binding region of the antibody or antibody fragment are selected from the group of: a single chain antibody (scFv), a recombinant camelid heavy-chain-only antibody (VHH), a shark heavy-chain-only antibody (VNAR), a microprotein, a darpin, an anticalin, and adnectin, an aptamer, an Fv, a Fab, a Fab', and a F(ab')2.

According to the application, the antibody or antibody fragment is provided in a dosage effective to decrease a symptom of a cancer or metastasis progression. For example, the symptom is selected from the group consisting of: size of primary tumor, growth of primary tumor, number of metastases, size of metastases; number of circulating tumor cells in blood, vascularization in tissue adjacent to the tumor or metastases, amount of ADAM8 in body fluids, amount of VEGF-A in body fluids, and weight loss of the subj ect.

According to the application, the therapeutically effective amount is formulated in a dosage within the range of about 0.1 mg to about 500 mg. In certain embodiments, the dosage is selected from about 0.1 mg, about 1 mg, about 5 mg, about 10 mg, about 25 mg, about 50 mg, about 75 mg, about 100 mg, about 125 mg, about 150 mg, about 175 mg, about 200 mg, about 250 mg, about 300 mg, about 400 mg, and about 500 mg.

According to the application, the unit dose is calculated from the weight of a subject in the range of about 0.01 mg/kg to about 200 mg/kg. For example, a unit dose is calculated from the weight of a subject in the range of about 0.1 mg/kg to about 10 mg/kg, or about 0.5 mg/kg to about 50 mg/kg, or about 1 mg/kg to about 100 mg/kg, or about 1.5 mg/kg to about 200 mg/kg, or about 0.5 mg/kg to about 1.5 mg/kg, or about 0.01 mg/kg to about 0.5 mg /kg. In various embodiments, the composition is administered on at least one regimen selected from the group consisting of: daily, weekly, biweekly, and alternating weeks

According to the application, the at least one antibody or antibody fragment is an immunoconjugate. Said immunoconjugate antibody or antibody fragment is bound to at least one selected from the group of a chemotherapeutic agent, a photosensitizing agent, a cytotoxic natural product, a phytotoxins, a cytotoxic antibiotic, a bacterial toxin, a fungal toxin, a bioactive protein, a radioisotype, an enzyme, and a combination of enzymes that activate an inert pre-cytotoxic agent.

According to the application, the therapeutically effective amount is formulated in a dosage selected from the group consisting of: about 1 mg, about 5 mg, about 10 mg, about 25 mg, about 50 mg, about 75 mg, about 100 mg, about 125 mg, about 150 mg, about 175 mg, about 200 mg, about 250 mg, about 300 mg, about 400 mg, and about 500 mg. For example, the therapeutically effective amount is formulated in a unit dose. In certain embodiments, the method according to claim 64, wherein the unit dose is calculated from the weight of the patient, wherein the dose/weight of the patient is a range from about 0.01 mg/kg to about 200 mg/kg.

In an aspect of the invention, the ectodomain of ADAM8 is a metalloproteinase domain (MP) and a disintegrin domain (DI).

In an aspect of the invention, the first ectodomain and the second ectodomain are the metalloproteinase domain (MP) and the disintegrin domain (DI). According to the invention the antibody or antibody fragment binds to an ADAM8 ectodomain and inhibits two ectodomain activities. According to the invention, the antibody or antibody fragment binds to the ADAM8 ectodomain and inhibits the metalloproteinase domain (MP) and the disintegrin domain (DI).

### Brief Description of the Drawings

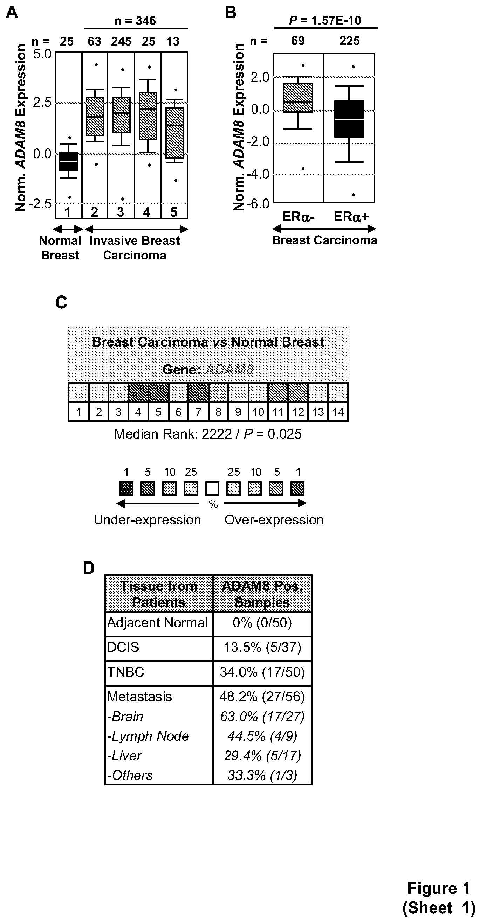

Figures 1A-1G show that ADAM8 is overexpressed in human breast cancer.
Figures 1A-1C are graphs and a table analyzing *ADAM8* mRNA expression in tumor samples from breast cancer patients using ONCOMINE™ microarray database. Figure 1A analyzes *ADAM8* mRNA expression which was observed to be significantly higher in invasive breast cancer samples compared to normal tissue (P<0.001). The cancer genome atlas (TCGA) dataset includes human normal breast (1) and invasive breast carcinoma: unknown origin (2), ductal (3), lobular (4), and mixed (5). Figure 1B analyzes the Van de Vijver data set which includes estrogen receptor (ER)α-negative and ERα-positive human primary breast carcinoma samples. n: sample size. Figure 1C pools 14 analyses from 6 different microarray studies and shows that *ADAM8* is one of the more highly expressed genes in breast cancer compared to normal breast tissue (P = 0.025).
Figures 1D-1F analyze ADAM8 protein expression in tumor samples from breast cancer patients by immunohistochemistry (IHC). IHC was used to measure ADAM8 staining analyzed in adjacent normal epithelial tissue (Adjacent Normal) and primary Triple-Negative Breast Cancer (TNBC) samples from 50 patients, in 37 samples of Ductal Carcinoma In Situ (DCIS), and in 56 samples of breast cancer-derived distant metastases.

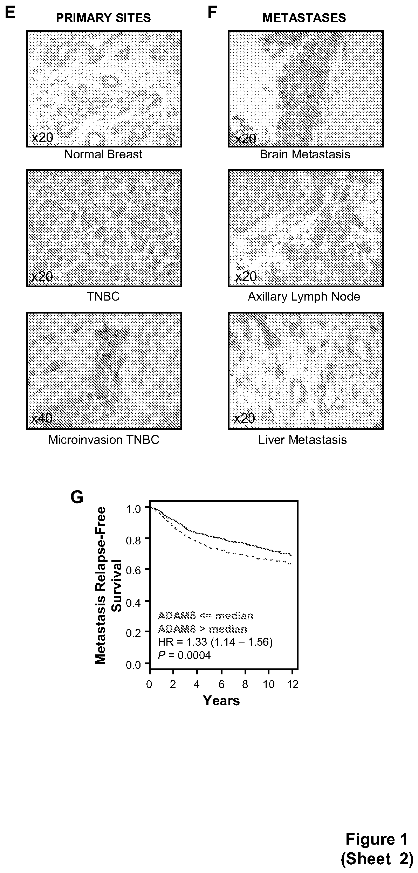

Figure 1D shows percentages of ADAM8-positive (Pos.) samples relative to the total analyzed for each group examined. Figure 1E shows representative pictures of ADAM8 staining in primary sites. Figure 1F shows representative pictures of staining for ADAM8 positive metastases. Figure 1G is a graph of prognostic value of *ADAM8* expression in breast cancer which was analyzed using the bc-GenExMiner microarray database. This analysis includes 2,314 patients with breast cancer and 628 events of metastatic relapse. High *ADAM8* mRNA levels were observed to correlate with a poorer metastasis relapse-free survival.

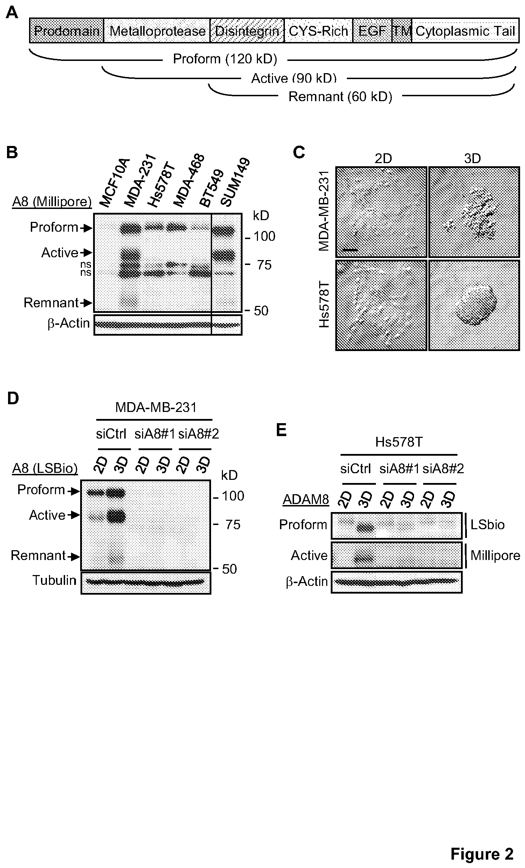

Figures 2A-2E are a diagram and sets of photographs showing that ADAM8 is abundantly expressed and active in TNBC cells.
Figure 2A is a schematic representation of ADAM8 protein showing the different domains and processed forms with molecular weights indicated. CYS-Rich: cysteine-rich domain, EGF: EGF-like domain, TM: transmembrane domain. Figure 2B shows whole cell extracts (WCEs) from human non-tumoral MCF-10A cells and the indicated TNBC cell lines examined by western blotting for ADAM8 expression, using ADAM8 Millipore antibody, and for β-Actin as a loading control. A representative blot is shown (n = 3). All lanes were from the same gel, but cut to re-align as indicated by the line. ADAM8 proform, active and remnant forms and molecular weight (MW) markers are indicated. ns: non-specific band. MDA-231: MDA-MB-231. MDA-468: MDA-MB-468. Figure 2C is a set of photomicrographs of cells grown in adherent (2D) or suspension cultures on ultra low-attachment plates (3D) for 48 h. In suspension, MDA-MB-231 and Hs578T cells form spheres. Scale bar: 100 µm. Figures 2D-2E show photographs of MDA-MB-231 (Figure 2D) and Hs578T (Figure 2E) cells reverse transfected with either *Control* siRNA (siCtrl) or two specific *ADAM8* siRNAs (siA8#1 and siA8#2) for 24 h, then plated for 48 h in 2D or 3D-culture as described in Figure 2C. WCEs were analyzed by western blot analysis for ADAM8 using only LSBio antibody (Figure 2D), or using both LSBio and Millipore antibodies (Figure 2E). As a loading control, tubulin (Figure 2D) or β-Actin (Figure 2E) was used. Representative blots from three independent examples are shown.

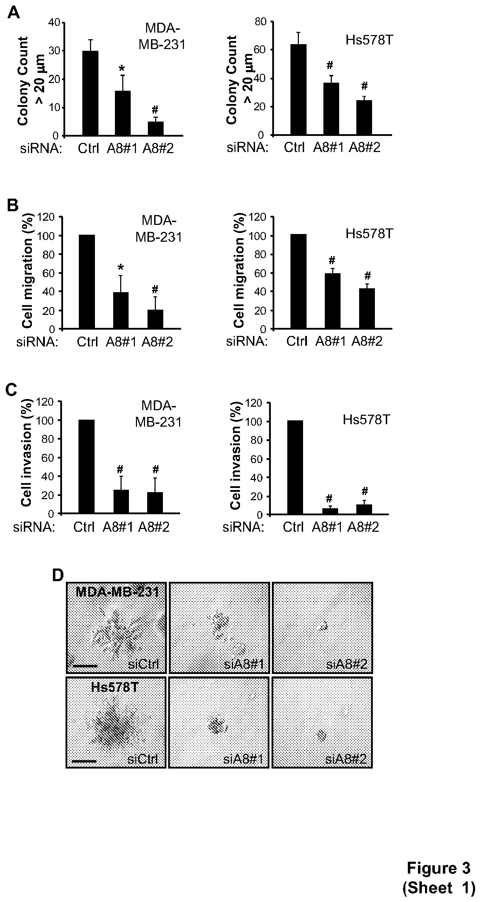

Figures 3A-3F show that ADAM8 maintains the aggressive phenotype of breast cancer cells in three-dimensional culture.

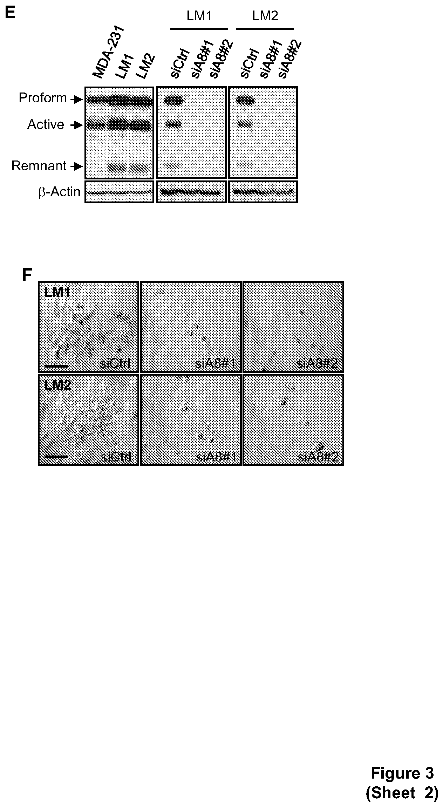

Figures 3A-3D are a set of bar graphs of data and photomicrographs of cells transfected with siRNAs as in Figures 2D-2E for 24 h, and tested for colony formation in soft agar (Figure 3A), cell migration (Figure 3B), invasion (Figure 3C), and 3D-Matrigel outgrowth (Figure 3D). For soft agar assays, cells were grown for 8-12 days, and colonies were stained with iodonitrotetrazolium violet and photographed. Colonies having a greater than 20 µm diameter in three wells/condition were counted with ImageJ software. A representative of two examples with similar results is shown (Figure 3A). Migration (Figure 3B) and invasion (Figure 3C) assays were performed using Transwells without or with precoating of Matrigel, respectively. After 24 h, cells that migrated to or invaded the lower side of the filter were evaluated by crystal violet staining and OD₅₇₀ₙₘ determination. Control condition (siCtrl) is set to 100% (mean ± standard deviation (S.D.) from three independent examples). Data obtained from Matrigel outgrowth assays are photographs at 20x magnification of colonies formed after 5-7 days (Figure 3D). Results shown were verified from duplicate assays. Figure 3E shows photographs of WCEs from MDA-MB-231 cells and *in vivo* derived LM1 and LM2 lines, either untransfected (left) or transfected with siCtrl, siA8#1 or siA8#2 for 48 h (right), and analyzed for ADAM8 by western blot analysis (LSBio antibody). Figure 3F shows photomicrographs of LM1 and LM2 cells analyzed by a 3D-Matrigel outgrowth assay 24 h after siRNA transfection, as in Figure 3D, in which colonies were photographed after 4 days. The assay was performed twice and similar results were obtained. Scale bars: 100 µm * *P* < 0.05, *# P* < 0.001, Student's t test.

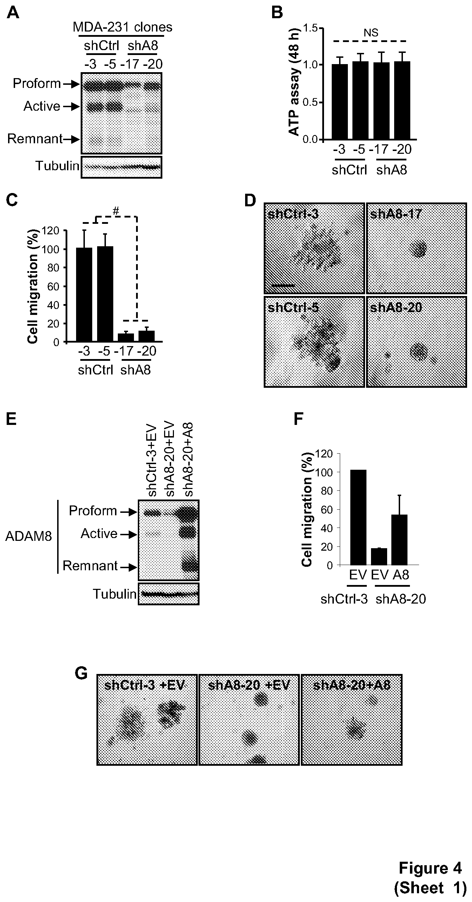

Figures 4A-4K are a set of photographs and graphs of data showing that ADAM8 knockdown profoundly decreases tumor formation in the mammary fat pad, CTC spread, and tumor metastasis.
Figure 4A is a western blot of stable ADAM8 knockdown (shA8) MDA-MB-231 clones that were characterized *in vitro.* ADAM8 expression in WCEs from two shA8 *(ADAM8* shRNA) clones (shA8-17 and shA8-20) was compared with two shCtrl clones (shCtrl-3 and shCtrl-5) using western blot analysis (LSBio antibody). Figure 4B is a bar graph of data from clones grown in two-dimensional cultures for 48 h and analyzed by ATP assay (n = 3 independent examples). NS: non-significant. Figure 4C is a bar graph of data from migration assays performed using Transwells as described in Figure 3B. Figure 4D is a set of photographs of Matrigel outgrowth assays performed as in Figure 3D. Scale bar: 100 µm.
Figures 4E-4G show data from a western blot, a migration assay, and a Matrigel outgrowth assay 24 hours after transfecting shA8-20 MDA-MB-231 cells with ADAM8 or an empty vector (EV) DNA. Figure 4E shows the results of a western blot analysis for ADAM8 (LSBio antibody) and tubulin in a whole cell extract. Figure 4F shows results of a migration assay performed as in Figure 3C. Figure 4G is a set of photographs of Matrigel outgrowth assays performed as in Figure 3D. Results in 4F and 4G show that ectopic expression of ADAM8 in shA8-20 cells was observed to partially rescue the cells' ability to migrate and to form invasive colonies.

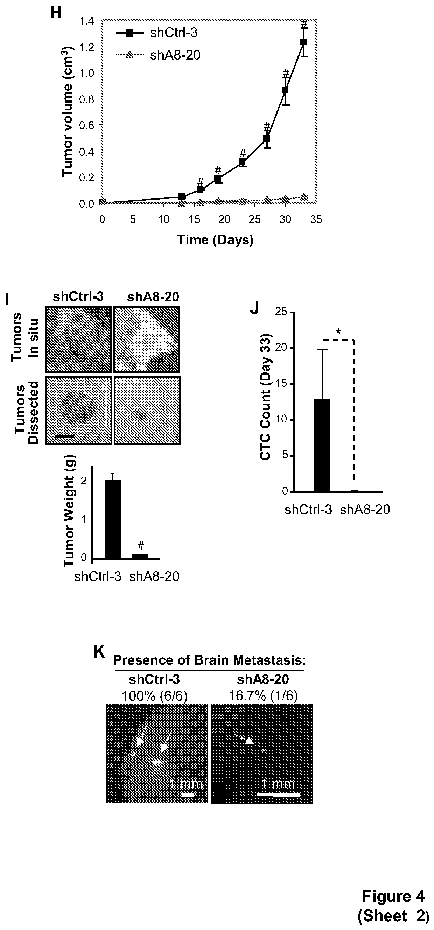

Figures 4H-4K show data from MDA-MB-231 derived cells shCtrl-3 and shA8-20 injected into the mammary fat pad of female NOD/SCID mice (n = seven/group). Figure 4H is a graph of tumor volume measured twice weekly (mean ± S.D.). Figure 4I shows tumors that were photographed *in situ* (upper) or after dissection (middle) and weighed at the end of the example (average weight ± S.D.) (bottom). Scale bar: 1 cm. Results show decreased size and poor vascularization of the shA8-20 compared to shCtrl-3 tumors. Figure 4J shows data from blood collected by cardiac puncture from which GFP-positive CTCs were detected by flow cytometry. The average CTC count ± S.D is given per µl of blood (n = seven/group). Figure 4K is a set of representative photographs showing presence of brain metastases examined by fluorescent microscopy (n = six/group). All of the control animals showed at least one metastasis, whereas a single micrometastasis was seen in only one shA8-20 mouse, and no metastases were observed in other shA8-20 mice. Scale bars: 1 mm. * *P* < 0.05, *# P* < 0.001, Student's *t* test.

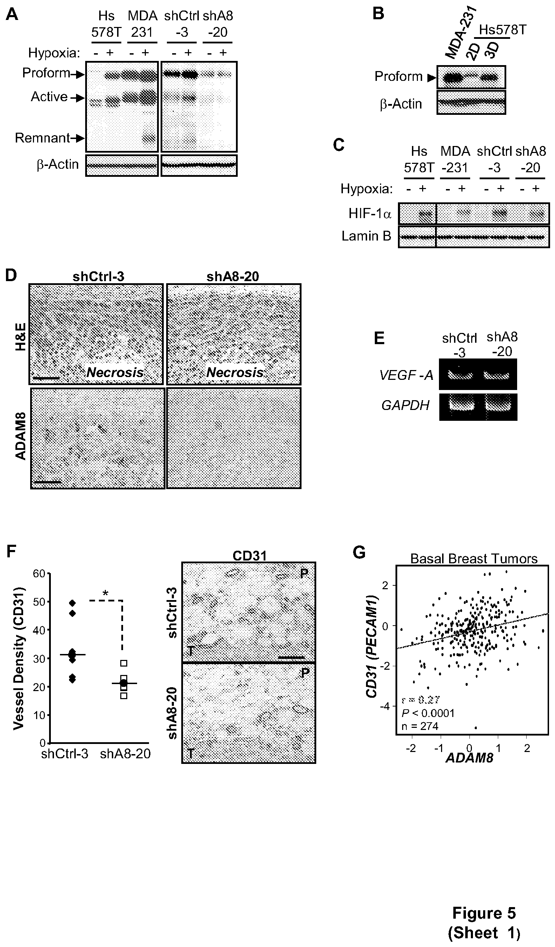

Figures 5A-5M show that ADAM8 is induced by hypoxia and promotes angiogenesis.

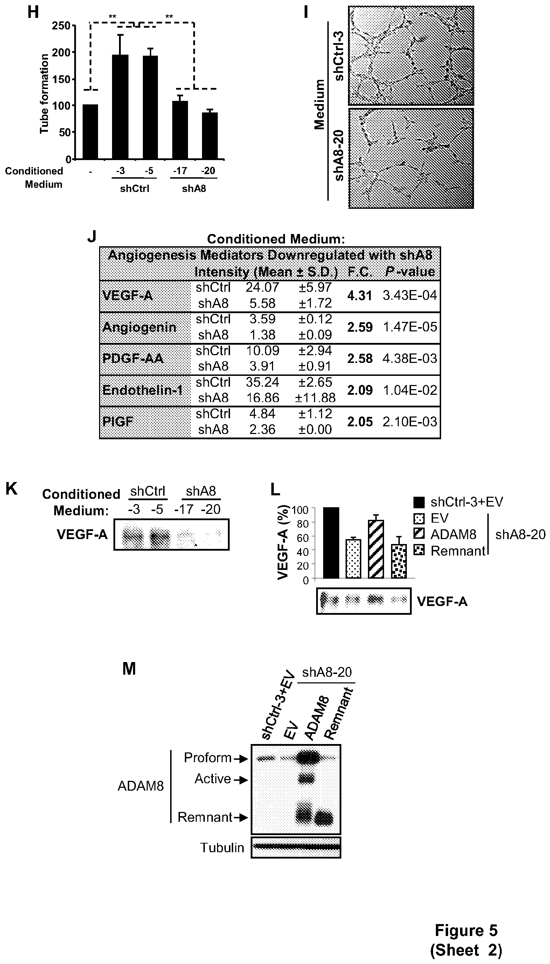

Figure 5A is a photograph of western blot analysis of WCEs of cells cultured under hypoxic (+, 1% O2) or normoxic (-) conditions for 24 hours (left) or 6 hours (right) and analyzed by western blot for ADAM8 (LSBio antibody). Representative blots from three independent examples are shown. Figure 5B is a photograph of western blot analysis of WCEs of Hs578T cells cultured in adherent conditions (2D) or on ultra low-attachment plates (3D) from suspension for 48 h. Figure 5C is photograph of western blot analysis of nuclear extracts from cells incubated for 8 hours under hypoxic conditions for HIF-1α and Lamin B. The vertical line indicates the two different times of exposure used: 5 minutes for Hs578T cells and 30 minutes for MDA-MB-231-derived lines. Figure 5D is a set of photographs showing ADAM8 expression in mouse mammary tumors analyzed by immunohistochemistry. H&E (hematoxylin and eosin) staining. Representative photographs are shown (n = seven/group). Figure 5E represents analysis of *VEGF-A* RNA amounts in shCtrl-3 and shA8-20 cells in culture which indicates no change in VEGF-A transcription with ADAM8 knockdown. Figure 5F is a graph and photographs showing angiogenesis evaluated by CD31 immunohistochemical staining of tumor sections from shCtrl-3 and shA8-20 groups (n = seven/group). Vessel density for each mouse is given as the average number of vessels observed in two slides/tumor (three peritumoral hot spots/slide). P: Peritumoral area, T: Tumor. Scale bars: 100 µm, * *P* < 0.05, ** *P* < 0.0001, Student's t test. Figure 5G is a Pearson's pairwise correlation plot showing a significant positive correlation observed between *ADAM8* and *CD31* (*PECAM1*) mRNA expression in tumors from patients with basal-like breast cancer which were obtained from GenExMiner microarray database. r: correlation ratio. *P* < 0.0001. Figures 5H-5I are a bar graph and photographs of Human Umbilical Vein Endothelial Cells (HUVECs) analyzed by tube formation assays in the presence of conditioned medium from the indicated shA8 and shCtrl clones, or obtained in absence of tumor cells (-). Values of closed networks are given as averages of nine fields ± S.D. **P < 0.0001, Student's *t* test (Figure 5H). Figure 5I shows representative images from the three independent examples shown in Figure 5H. Figure 5J is a table showing the expression levels of angiogenesis mediators in conditioned media from two shCtrl and two shA8 clones that were analyzed by a Human Angiogenesis antibody array. Expression levels of the detected proteins were quantified using ImageJ. The table shows that the angiogenesis mediators were significantly downregulated by more than two-fold in shA8 clones (range of 2.05-fold to 4.31-fold). Data presented as mean of the two clones ± S.D. Fold change (F.C.) and P-values are given. Figure 5K is a photograph of a western blot analysis (n=3) of conditioned serum-free medium from shCtrl and shA8 clones analyzed for VEGF-A protein levels. Figure 5L is a photograph of western blot analysis showing VEGF-A protein levels in the conditioned serum-free medium from shCtrl-3 or shA8-20 clones transfected with the indicated ADAM8 forms (complete or remnant) or empty vector (EV) DNA (lower), and a bar graph of quantification of average levels from three experiments as percent relative to the shCtrl set to 100% (upper). Figure 5M is a photograph of western blot analysis of the amount of ectopic expression of full-length ADAM8 and remnant form in stable ADAM8 knockdown cells.

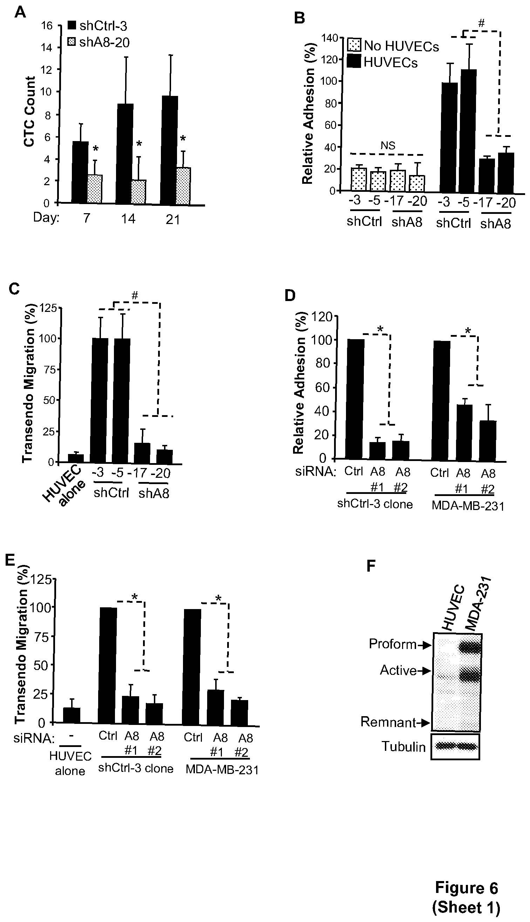

Figures 6A-6J show that ADAM8 promotes cancer cell adhesion to endothelial cells and β1-integrin activation.

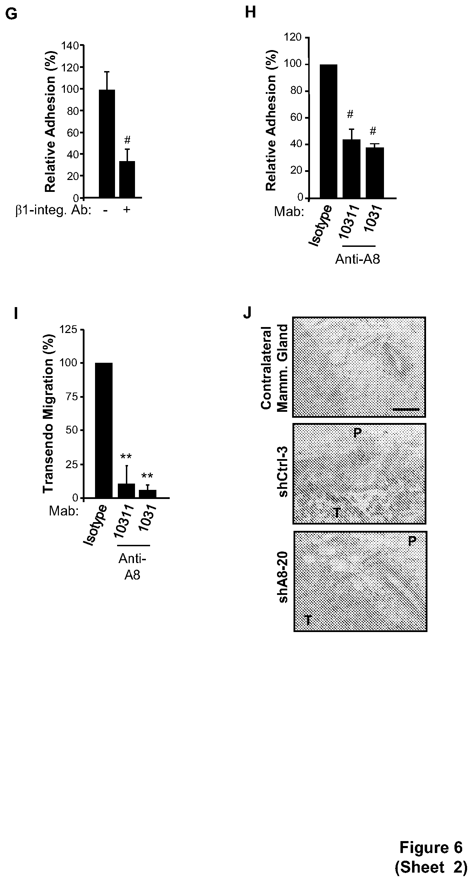

Figure 6A is a bar graph of data from blood that was drawn using the submandibular method from mice (n = four/group) on the indicated days after tumor cell implantation and analyzed by flow cytometry to measure GFP-positive CTCs. The average CTC count ± S.D. is given per µl of blood. Figure 6B is a bar graph showing relative adhesion (%) of cells incubated either on a HUVEC monolayer or in empty wells (No HUVECs) for 15 minutes. Attached cells were counted in three fields per well (n = 6 fields). Mean ± S.D. from three independent examples is shown. Figure 6C is a bar graph of data from shADAM8 and shControl clones analyzed by an overnight transendothelial migration assay on HUVEC cell monolayers. Mean ± S.D. from three independent examples is shown. Figure 6D is a bar graph that shows relative adhesion (%) to HUVECs of MDA-MB-231-derived shCtrl-3 clone cells and MDA-MB-231 cells that had been transfected with either Control siRNA (siCtrl) or two ADAM8 siRNAs (siA8#1 and siA8#2) for 72 h. Figure 6E is a bar graph of the transendothelial cell migration assay of the cells described in Figure 6D through HUVECs. **P* < 0.05, Student's t test. Figure 6F is a photograph of western blot analysis for ADAM8 in WCEs from HUVECs and MDA-MB-231 as a positive control for ADAM8 expression. Figure 6G is a bar graph of adhesion of shCtrl-3 cells on HUVECs assessed as in Figure 6B with prior incubation of shCtrl-3 cells with an antagonist β1-integrin antibody (+) or a control isotype (-). Mean ± S.D. from three independent experiments. Figure 6H and 6I are bar graphs showing the results of adhesion (Figure 6H) and transendothelial migration (Figure 6I) assays of shCtrl-3 cells on HUVECs assessed as in Figures 6B and 6C, respectively. Prior to the assays, shCtrl-3 cells were incubated either with monoclonal antibodies targeting the ectodomain of ADAM8 (Mab10311 specific for the CRD/ELD or Mab1031 specific for the MP/DI domains) or an appropriate isotype control (IgG1 or IgG2B, respectively). The transmigration assay was performed for 9 h. Mean ± S.D. from three independent experiments. Figure 6J is a set of photographs showing active β1-integrin expression assessed by immunohistochemistry (IHC) in mouse mammary tumors (shCtrl-3 and shA8-20) and contralateral mammary glands (shCtrl-3; n = seven/group). P: Peritumoral area, T: Tumor. Bar: 100 µm. NS, non-significant, * *P* < 0.05, *# P* < 0.001, ***P* < 0.0001, Student's t test.

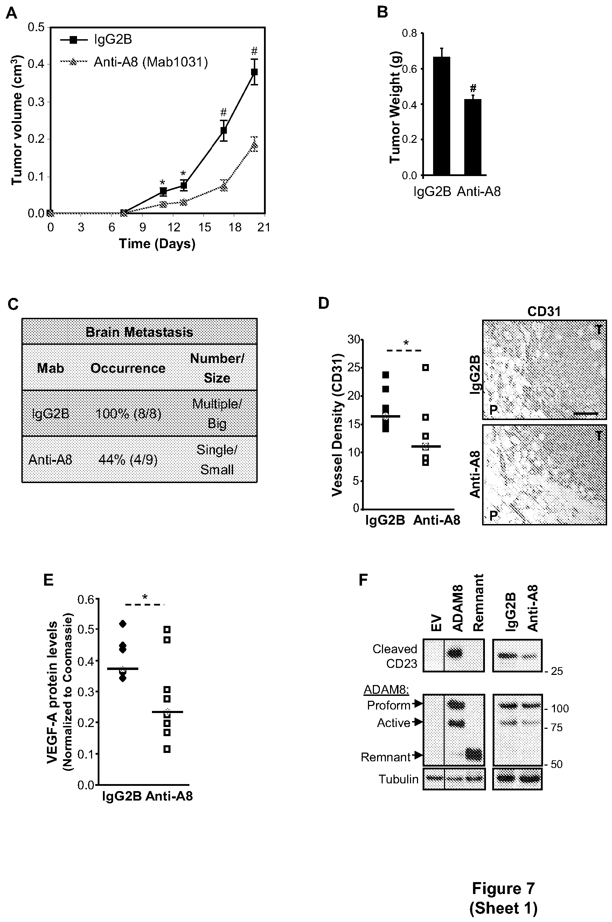

Figures 7A-7H are a set of graphs, photographs, a table, and a drawing showing that ADAM8 monoclonal antibody decreases orthotopic tumor formation, angiogenesis and metastasis. Figures 7A-7H were performed with MDA-MB-231 shCtrl-3 cells injected into the mammary fat pads of female NOD/SCID mice. Animals were treated with either 0.5 mg/kg anti-ADAM8 (anti-A8, Mab1031, n = 9) or isotype-matched control (IgG2B, n = 8) in intraperitoneal (i.p.) injection twice weekly from the time of cell implantation.

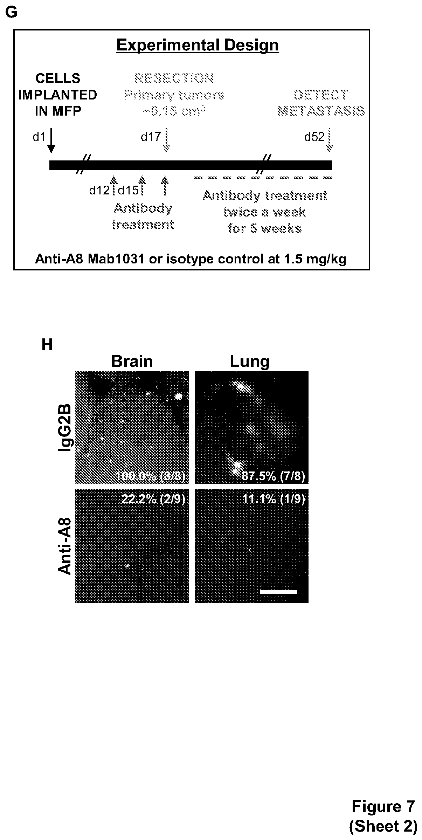

Figure 7A is a line graph of tumor volume measured twice a week (mean ± S.D.). Figure 7B shows tumor weight at the end of the time course (mean ± S.D.). Figure 7C is a table summarizing occurrence, number, and size of brain metastases detected by fluorescent microscopy at the end of the time course. Figure 7D shows angiogenesis evaluated by CD31 immunohistochemical staining of tumor sections. Vessel density for each mouse is shown on the ordinate (left), determined as the average number of vessels in two slides/tumor (five peritumoral hot spots/slide), and representative pictures (right). P: Peritumoral area, T: Tumor. Bar: 100 µm. Figure 7E is a graph showing VEGF-A levels in the tumor extracts determined by western blot and normalized to Coomassie staining. **P* < 0.05, *^{#}P* < 0.001, Student's t test. Figure 7F is a photograph of western blot analysis of the amount of cleaved CD23 (29kDa) in the medium of HEK293 cells that were co-transfected with vectors expressing CD23 and the indicated forms of ADAM8. Cells were harvested and WCEs were assessed for expression of ADAM8 and tubulin. Figure 7G is a drawing of a scheme of the experimental design used to evaluate the therapeutic effect of the anti-ADAM8 antibody (Mab1031) on metastasis in a clinically relevant model. Figure 7H is a set of photographs of metastases to the brain and lungs detected by fluorescent microscopy. Representative images and frequency of metastases (percentage of animals positive per group: IgG2B, n = 8; anti-A8, n = 9) are presented. Bar: 250 µm.

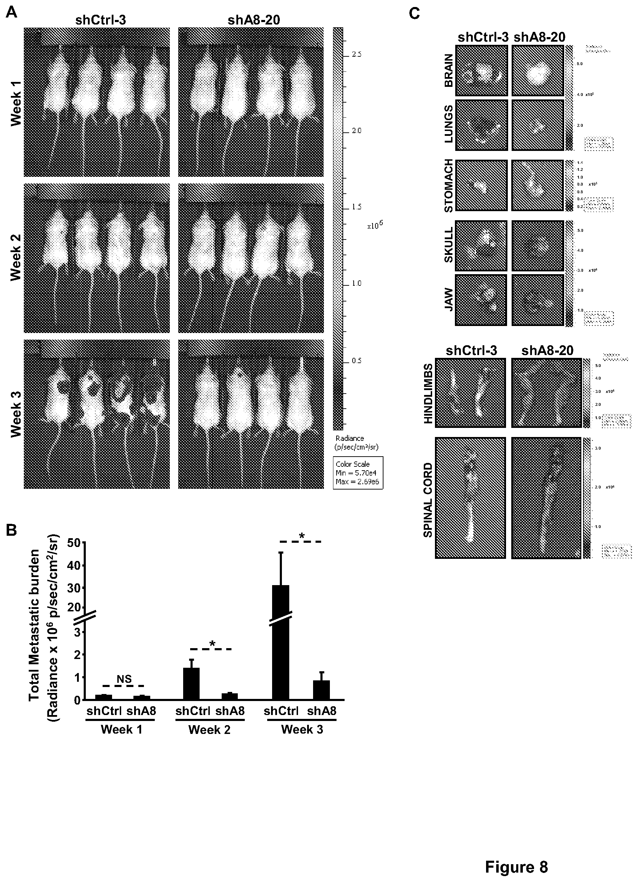

Figure 8 is a set of photographs and a bar graph of data showing that ADAM8 promotes metastasis in a cardiac colonization mouse model.
Figure 8A is a set of photographs of female mice which were injected in the left ventricle (n = four/group) with MDA-MB-231 derived shCtrl-3 and shA8-20 cells, which stably express Luciferase. Tumor burden was measured weekly for three weeks by detection of Luciferase activity using the Xenogen Biophotonic Imaging system. Figure 8B shows measured Luciferase activity corresponding to total metastatic burden as mean ± S.E. per group for each week. Figure 8C shows organs and bones harvested at the end of the experiment and imaged as described above. NS: non-significant, *P < 0.05, Mann-Whitney U test.

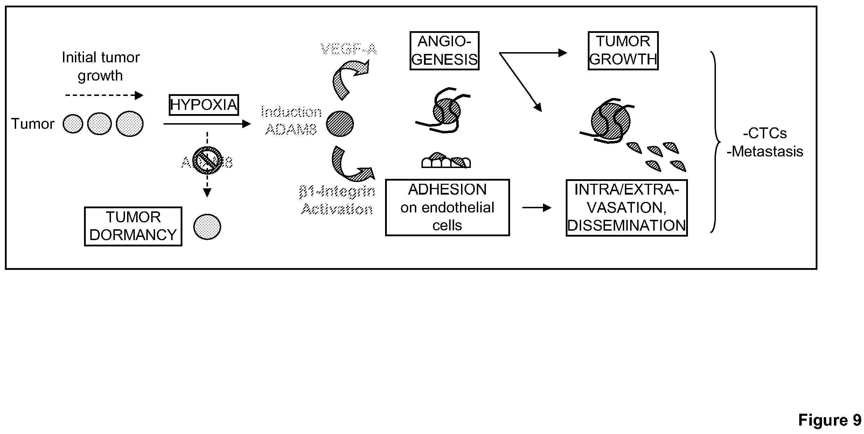

Figure 9 is a drawing of a model of functions of ADAM8 in breast tumor growth and dissemination, envisioned from data observed in examples. When solid tumors reach a few millimeters in diameter, hypoxic stress is induced, which leads to endothelial cell recruitment and angiogenesis. Data herein show that under conditions in which ADAM8 was not induced, there was an insufficient angiogenic response, which leads to tumor mass dormancy. When ADAM8 is induced in tumor cells by hypoxia, strong pro-angiogenic signaling, in part through metalloproteinase (MP) activity leading to VEGF-A released into the extracellular compartment prevents angiogenic dormancy and sustains tumor growth. ADAM8 through its disintegrin (DI), cysteine-rich domain (CRD), and EGF-like domain (ELD) further promotes β1-integrin activation on tumor cells needed for adhesion onto and transmigration through the blood vessel wall, which supports dissemination of CTCs and metastasis.

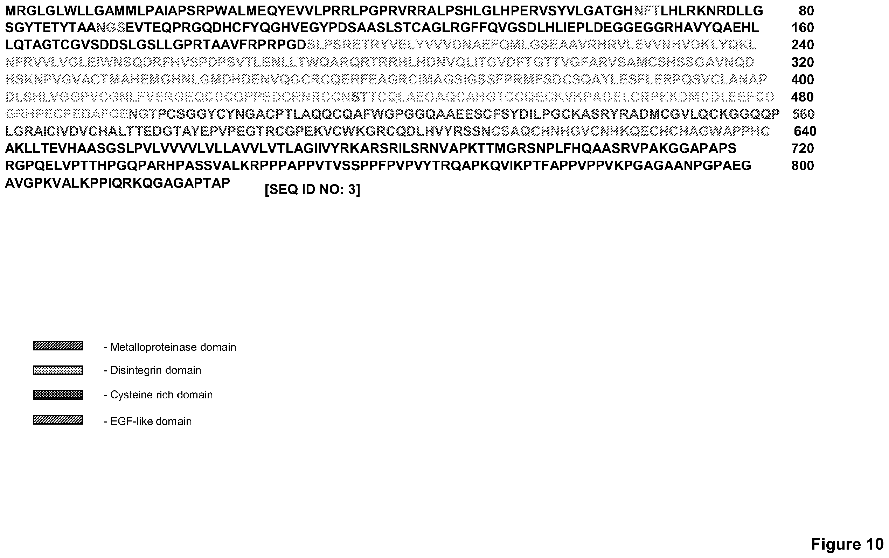

Figure 10 shows the amino acid sequence of human ADAM8 protein using the one letter amino acid code (SEQ ID NO: 3).

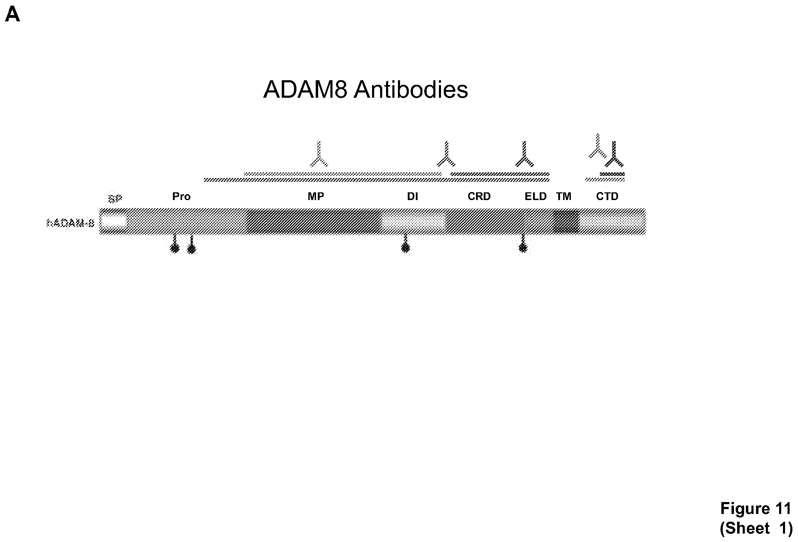

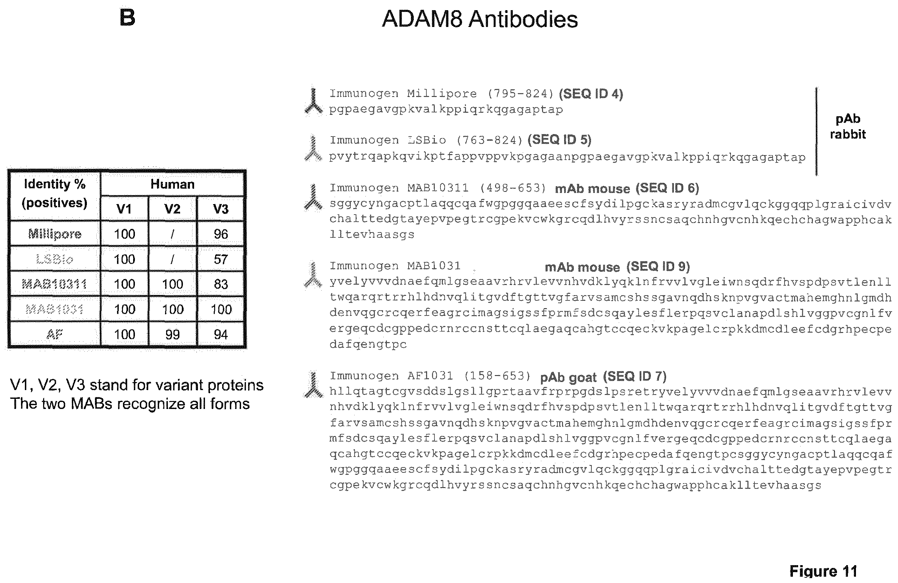

Figure 11 shows the amino acid sequences of anti-ADAM8 protein antibody immunogens for antibody preparations used herein. Millipore AB19017 was prepared with a peptide immunogen having residues 795-824 of the ADAM8 protein (SEQ ID NO: 4). LSBio LS-B4068 was prepared with a peptide immunogen having residues 763-824 of the ADAM8 protein (SEQ ID NO: 5). MAB10311 was prepared with a peptide having residues 498-653 of the ADAM8 protein (SEQ ID NO: 6). MAB1031 was prepared with a peptide immunogen having residues 201-498 (SEQ ID NO: 9) of the ADAM 8 protein. AF1031 was prepared with a peptide immunogen having residues 158-653 of the ADAM8 protein (SEQ ID NO: 7). The top bar shows the positions of the peptide domains that function as immunogens for preparation of Millipore AB19017, LSBio LS-B4068 , MAB10311, MAB1031 and AF1031 relative to one another. The markers indicate the position of N-glycosylation of the ADAM8 protein. The table side shows the percentage of positive identifications of the three human ADAM8 protein variants by each antibody.

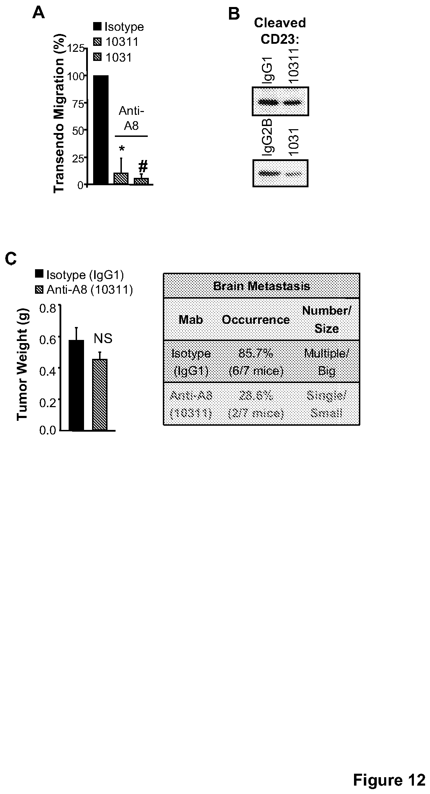

Figures 12A-12C show that metastasis is decreased in brain tissue by administration of a monoclonal antibody that specifically binds to the cysteine-rich domain (CRD) and the epidermal growth factor-like domain (ELD).
Figure 12A shows migration of shCtrl-3 breast cancer cell lines using a transmigration assay. Transendothelial cell migration through HUVECS was analyzed after treatment with isotype control IgG1, isotype control IgG2B, monoclonal antibody Mab10311 that specifically binds to CRD and ELD of ADAM8 (anti-ADAM8 CRD/ELD), and monoclonal antibody Mab1031 (anti-ADAM8 MP/DI) that specifically binds to MP and DI domains of ADAM8. Each isotype was normalized to 100%. Cell migration of the Mab10311treated group was compared to the group treated with IgG1, and cell migration of the Mab1031 treated group was compared to the group treated with IgG2B. The sample treated with anti-ADAM8 CRD/ELD and the sample treated with anti-ADAM8 MP/DI had significantly less cell migration through the HUVEC monolayer than the sample treated with the appropriate isotype control.
Figure 12B is a photograph of western blot analysis of the amount of cleaved CD23 in HEK293 cells that had been cotransfected with CD23 and ADAM8 vectors and treated with anti-ADAM8 CRD/ELD or anti-ADAM8 MP/DI compared to treatment with IgG1 or IgG2B. Results show that in the presence of anti-ADAM8 CRD/ELD, cleavage of CD23 was modestly reduced compared to in the presence of anti-ADAM8 MP/DI, which indicates that the anti-ADAM8 MP/DI antibody more robustly inhibits MP activity.
Figure 12C shows the effect of treatment with anti-ADAM8 CRD/ELD on primary tumor weight and metastasis from the mammary fat pad (MFP) of female mice. shCtrl-3 cells were injected into the MFP, then each mouse was treated with either anti-ADAM8 CRD/ELD or isotype-matched control IgG1. The left graph analyzes the differences in primary tumor weight using a Students *t*-test after treatment. Results show that the differences in primary tumor weight between mice treated with anti-ADAM8 CRD/ELD and mice treated with IgG1 were not statistically significant (NS). The right photograph analyzes brain metastasis using fluorescent microscopy after treatment with either anti-ADAM8 CRD/ELD or IgG1. Results show that treatment with anti-ADAM8 CRD/ELD greatly reduced both size and frequency of brain metastasis.

### Detailed Description

ADAM8 belongs to a large family of proteins consisting of more than 30 members. Synthesized as a proform, ADAM8 can dimerize or multimerize and autocatalytically clip off its prodomain, leaving an active membrane-anchored metalloproteinase (Figure 2A). Active ADAM8 can be further processed by the release of the metalloproteinase domain into the extracellular matrix, which leaves a remnant form within the membrane. ADAM proteins share similarity in overall structure, however the cysteine-rich domain contains a hypervariable region that allows for differentiation between family members and has been found to be critical for function of ADAM proteins. There is an important site of glycosylation between the EGF-like and cysteine-rich domains.

An aspect of the invention provides a composition, for example, an antibody directed against the ADAM8 ectodomain region that further inhibits growth and/or spreading of cancer cells in patients with breast tumors that express ADAM8. In particular, an antibody against a region spanning part or all of the metalloproteinase/disintegrin domains will be effective. The term "against" as used herein refers to antibody specificity, such that an antibody against a polypeptide has affinity to recognize and bind to that peptide. Furthermore, as approximately half of breast cancer-derived metastases displayed ADAM8 expression, ADAM8 or downstream signaling molecules are useful as biomarkers for disease progression. Identifying new therapeutic targets to effectively block breast cancer dissemination is crucial to improve the overall survival of patients at high risk, e.g., women with triple-negative breast tumors. Since ADAM8 is expressed also in other human cancers, including brain, lung, prostate, skin, colorectal, ovarian, bone, renal, head and neck, esophageal, gastric, liver, bladder, cervical, testicular, uterine, thyroid, and pancreatic cancers, inhibitors of ADAM8 should also be effective in the treatment of patients with these types of tumors. Further, ADAM8 or downstream signaling molecules can be used as biomarkers for diagnosis of these cancers.

ADAM8 is a transmembrane protein that belongs to the ADAM (a disintegrin and metalloproteinase) family and mediates cell adhesion, cell migration, and proteolysis of a variety of substrates, including cytokine receptors or ligands, cell adhesion molecules and extracellular matrix components (Koller et al. Curr Pharm Des 15:2272-2281, 2009). Synthesized as a proform, ADAM8 can dimerize or multimerize and autocatalytically clip off its prodomain, leaving an active membrane-anchored metalloproteinase (Figures 2A and 11). Active ADAM8 can be further processed by the release of the metalloproteinase domain into the extracellular matrix, which leaves a remnant form within the membrane. Both active and remnant forms of ADAM8 mediate cell adhesion through disintegrin/cysteine-rich/EGF-like domains (Schlomann et al. J Biol Chem 277:48210-48219, 2002), notably by direct binding to integrins (Rao et al. J Bone Miner Res 21:1657-1665, 2006).

ADAM8 was found to be non-essential under physiological conditions, as evidenced by the normal development and lack of pathological defects in ADAM8 deficient mice (Kelly et al. Dev Dyn 232:221-231, 2005), despite its expression in a variety of immune cells (Richens et al. Immunobiology 212:29-38, 2007 and Yoshiyama et al. Genomics 41:56-62, 1997). ADAM8 expression was detected under several pathological conditions characterized by inflammation and extracellular matrix remodeling, including cancer (Koller et al. Curr Pharm Des 15:2272-2281, 2009). Using microarray analysis, ADAM8 was identified as a target of a multistep pathway downstream of NF-κB RelB, which is known to promote an aggressive phenotype in breast cancer (Wang et al. Nat Cell Biol 9:470-478, 2007). Analysis herein of publically available microarray databases indicated that *ADAM8* is expressed in aggressive breast cancers, including TNBC, and correlated with a poor patient outcome (Figure 1). Accordingly, the Examples described herein sought to determine the role of ADAM8 in breast cancer.

Data herein show that ADAM8 is required to maintain the aggressive phenotype of TNBC cells in three dimensional (3D) culture, and promotes angiogenesis, spread of CTCs, and metastatic dissemination in orthotopic breast and cardiac mouse models. The term "orthotopic" as used herein conventionally refers to a disease model affecting the same tissue and location as in the original disease.

Using the orthotopic models herein, ADAM8 was found to induce both vascular remodeling by the release of soluble pro-angiogenic factors, including VEGF-A (vascular endothelial growth factor A), and adhesion and transmigration through the endothelium following β1-integrin activation. Overall, these findings indicate that the transmembrane ADAM8 protein constitutes a therapeutic target for treatment of aggressive breast tumors.

Results herein from analyzing samples obtained from publically available cancer microarray datasets, show that *ADAM8* mRNA levels were significantly elevated in breast cancer in comparison to normal breast tissue, and more specifically in aggressive subtypes including estrogen receptor-negative breast tumors. Furthermore, immunohistochemistical analysis of a cohort of patients with triple-negative breast tumors (negative for estrogen receptor, progesterone receptor, and HER2) revealed that 34% (17/50) were positive for ADAM8 protein, and that adjacent normal tissues were negative. Importantly, 48% (27/56) of all breast tumor metastases in the samples were positive for ADAM8. Consistently high *ADAM8* mRNA levels were observed to be significantly correlated with poor metastasis relapse-free survival.

ADAM8 was tested herein within breast cancer cells in culture, and data show that this protein plays an essential role in migratory and invasive properties of cancer cells. In particular, ADAM8 promoted formation of invasive branching colonies and anchorage-independent growth in 3D assays of TNBC cells. An orthotopic xenograft mouse model was used to show that tumors derived from MDA-MB-231 TNBC cells in which ADAM8 was knocked down by a specific shRNA, failed to grow beyond a palpable size. In contrast, tumors derived from shControl MDA-MB-231 cells grew to an average size of 1 cm³ over the time course. ADAM8 was observed to be induced in breast cancer cells cultured under hypoxic conditions. High ADAM8 expression *in vivo* was detected around necrotic areas within tumors derived from shControl MDA-MB-231 cells but not in those from shADAM8 cells. shADAM8 tumors were unable to induce angiogenesis and hence remained dormant. Endothelial vessel density was assessed by immunohistochemical staining for the angiogenic marker CD31 in both tumor groups. CD31 staining revealed that vessel density (angiogenesis) was significantly decreased at the peritumoral area of shADAM8 compared to the shControl MDA-MB-231 cell-derived tumors. All animals injected with shControl cells were positive for brain macrometastases. In contrast, only one mouse had a small metastasis in the shADAM8 group.

To determine the mechanism by which ADAM8 induces angiogenesis, the effect of conditioned medium obtained from MDA-MB-231 cells on ability of Human Umbilical Vein Endothelial Cells (HUVECs) to form vessel-like structures (tube formation) was tested. Conditioned media from shControl MDA-MB-231 cells poorly promoted tube formation. Conditioned media from shADAM8 MDA-MB-231 did not promote tube formation. Furthermore, release of Vascular Endothelial Growth Factor (VEGF)-A and four other angiogenesis mediators was observed to be reduced in the conditioned medium obtained from shADAM8 MDA-MB-231 cells compared to shControl cells using an angiogenesis protein array. Further, the metalloproteinase domain of ADAM8 was found to be required for the release of VEGF-A.

Binding of primary tumor cells and then circulating tumor cells (CTCs) to an endothelial layer followed by transmigration of the CTCs through the blood vessel wall (intravasation and extravasation, respectively) are critical steps in metastasis. ADAM8 knockdown was observed to reduce binding of MDA-MB-231 cells to HUVECs in culture, i.e., shControl cells were found to adhere much more efficiently than shADAM8 cells to a confluent HUVEC monolayer. Furthermore, in a transendothelial cell migration (TEM) assay, shADAM8 cells displayed a profoundly decreased ability to migrate through a confluent HUVEC monolayer in comparison to shControl cells. Thus, ADAM8 promotes tumor cell adhesion onto an endothelial layer, as is found in a vessel wall, and migration through the vessel wall.

The role of ADAM8 involvement in the modulation of β1-integrin activation, a critical step in adhesion of cancer cells to the endothelium, was investigated. Pre-treatment of shControl cells with an antagonist antibody targeting β1-integrin greatly reduced the ability of the cells to bind to HUVECs. Monoclonal antibodies targeting either the cysteine-rich/EGF-like or the metalloproteinase/disintegrin extracellular domains of ADAM8, caused inhibition of adhesion of shControl cells onto HUVECs essentially to the same extent as the β1-integrin antibody, and similarly prevented transmigration of the cells in a TEM assay.

Examples herein show that mice with shADAM8 tumors had fewer CTCs compared to those with shControl tumors, as revealed by analysis of blood samples. The result is consistent with a role for ADAM8 in adhesion and migration of cancer cells from the primary tumor site through induction of angiogenesis and interactions with the endothelium. This effect was observed even at early time points after injection of tumor cells into the mouse mammary fat pad when tumors from both groups were barely palpable and did not show any significant difference in size.

To validate ADAM8 as a therapeutic target for TNBC, the Mab1031 ADAM8 antibody targeting the MP/DI domains was selected for use in an orthotopic model.

Mice were treated with either 0.5 mg/kg of Mab1031 antibody (n = 9) or with this dose of a control isotype-matched IgG2B (n = 8) by i.p. injection twice weekly from the day of implantation of shCtrl-3 cells. Anti-ADAM8 treatment was observed to result in a significantly decreased primary tumor burden by about 50% (Figure 7A) and tumor weight by about 36% (Figure 7B) after three weeks of treatment. In animals treated with anti-ADAM8, a substantial reduction was seen also in the size and numbers of brain metastases per mouse, and in the frequency of brain metastases (Figure 7C). Angiogenesis surrounding the tumors (Figure 7D) and VEGF-A levels in the tumors (Figure 7E) were similarly reduced by treatment with the ADAM8 antibody. The ADAM8 metalloproteinase substrate CD23 (Koller et al. Curr Pharm Des 15:2272-2281, 2009) was used in an *in vitro* analysis of MP activity. ADAM8 activity resulted in a substantial reduction in the *in vitro* activity, which indicates inhibition of metalloproteinase in the presence of an anti-ADAM8 antibody (Figure 7F).

To test potential use of this ADAM8 antibody in a more clinically relevant system, a tumor resection model was used (Figure 7G). Ability to treat pre-existing tumors with Mab1031 anti-ADAM8 antibody was tested.

Mice were injected in the MFP with MDA-MB-231 shCtrl-3 cells, and palpable tumors were observed 12 days later. Treatment was initiated with 1.5 mg/kg of Mab1031 anti-ADAM8 antibody or isotype control IgG2B. Additional doses of antibody were administered on day 15 and on day 17, at which point tumors reached a volume of about 0.15 cm³ and tumor resection was performed (Figure 7G). Treatment with the anti-ADAM8 antibody or control IgG2B was continued twice weekly for a 5-week period, and then metastasis in the brain and lungs was examined using fluorescent microscopy (Figure 7H).

In the control group, metastases to the brain and lungs were seen in almost all of the subjects (8/8 and 7/8), respectively. In contrast in the anti-ADAM8-treated group, only 2/9 and 1/9 mice showed metastases in the brains and lungs, respectively. Thus, treatment with an anti-ADAM8 MP/DI antibody resulted in a profound reduction in frequency of metastases to each of the brain and lungs compared to the frequency of metastases in the isotype control antibody treated group.

To test the role of ADAM8 in the ability of breast cancer cells to colonize typical distant metastasis sites, MDA-MB-231-derived shCtrl-3 and shA8-20 cells, which stably express Luciferase, were injected in the left ventricle of female NOD/SCID mice (n = 4/group). Total tumor burden was monitored weekly by Luciferase activity measurement using the Xenogen Biophotonic Imaging system. Organ and bone metastases were assessed at sacrifice using this method.

Cardiac injection of MDA-MB-231-derived shA8-20 cells resulted in a significantly reduced total tumor burden after 2 and 3 weeks compared to injection of shCtrl-3 cells (Figure 8A-B). At the termination of the example, shCtrl-3 cells were consistently found to have colonized numerous organs and bones, in contrast to the shA8-20 cells (Figure 8C). Thus, ADAM8 was observed to have been required for colonization of distant metastasis sites by TNBC cells.

Since both the Mab10311 (CRD/ELD) and the Mab1031 (MP/DI) antibodies reduced amount of transendothelial cell migration to about the same extent (Figures 6I and 12A), targeting of the CRD/ELD region of ADAM8 was tested for inhibition of tumor growth and dissemination of TNBC in an orthotopic model. The abilities of each of the Mab10311 (CRD/ELD) antibody and the Mab1031 (MP/DI) antibody to inhibit the MP activity of ADAM8 were compared.

The Mab10311 antibody was observed to have inhibited the MP activity about 40%, a lesser extent than Mab1031, 70%. (Figure 12B) The Mab10311 ADAM8 antibody was then tested in the orthotopic model. Mice were treated with 1.5 mg/kg of either Mab10311 antibody (n = 7) or a control isotype-matched IgG1 (n = 7) by i.p. injection twice weekly from the day of implantation of shCtrl-3 cells.

After three weeks, no significant change in primary tumor burden was observed as a result of treatment with anti-ADAM8 Mab10311 (Figure 12C, left bar graph). However, a striking reduction in the size and numbers of brain metastases per mouse and in the overall frequency of metastases was observed in animals treated with anti-ADAM8 Mab10311 antibody (Figure 12C, right chart). These results indicate that inhibition of the DI activity of ADAM8 by interaction of an antibody with CRD/ELD regions was sufficient to profoundly reduce TNBC tumor dissemination. These data further indicate that inhibition of the MP activity, which is required for release of pro-angiogenic factors, is necessary to prevent tumor growth. Thus, inhibition of both the MP and DI activities of ADAM8 was observed to be required to prevent both tumor growth and dissemination.

A portion of the invention herein has been published in a paper entitled, "ADAM8 expression in invasive breast cancer promotes tumor dissemination and metastasis", EMBO Molecular Medicine 6(2): 278-294,2014, published online Dec. 27, 2013, by coauthors Mathilde Romagnoli, Nora D. Mineva, Michael Polmear, Catharina Conrad, Srimathi Srinivasan, Delphine Loussouarn, Sophie Barillé-Nion, Irene Georgakoudi, Áine Dagg, Enda W. McDermott, Michael J. Duffy, Patricia M. McGowan, Uwe Schlomann, Maddy Parsons, Joerg W. Bartsch, and Gail E. Sonenshein.

The invention having now been fully described is exemplified by the following examples and claims, which are exemplary and are not to be construed as further limiting.

### Antibodies

The present invention relates to compositions, methods, and kits for prognosing, diagnosing and treating ADAM8-related cancer conditions. The term "antibody" as used herein includes whole antibodies and any antigen binding fragment (i.e., "antigen-binding portion") or single chains of these. A naturally occurring "antibody" is a glycoprotein including at least two heavy (H) chains and two light (L) chains inter-connected by disulfide bonds, or is a camelid antibody having a single chain.

As used herein, an antibody that "specifically binds" a target protein or cell is intended to refer to an antibody that binds to a domain or portion of the target having an amino acid sequence. For example, the antibody has a K_{D} of 5 x 10⁻⁹ M or less, 2 x 10⁻⁹ M or less, or 1 x 10⁻¹⁰ M or less. For example, the antibody is monoclonal. Alternatively, the antibody is polyclonal. The terms "monoclonal antibody" or "monoclonal antibody composition" as used herein refer to a preparation of antibody molecules of single molecular composition. A monoclonal antibody composition displays a single binding specificity and affinity for the target or for a particular epitope of target. The antibody is an IgM, IgE, IgG such as IgG1 or IgG2b, and IgA.

Also useful for systems, method, and kits herein is an antibody that is recombinantly produced. The term "recombinant human antibody", as used herein, includes all anti-ADAM8 or control antibodies that are prepared, expressed, created, or isolated by recombinant means, such as antibodies isolated from human genes expressed in an animal (e.g., mouse, pig, goat, rabbit, sheep, primate, monkey, dog, and high value animals). Mammalian host cells for expressing the recombinant anti-ADAM8 antibodies used in the methods herein include Chinese Hamster Ovary (CHO cells) including dhfr-CHO cells and NSO myeloma cells, (Urlaub and Chasin, Proc. Natl. Acad. Sci. USA 77:4216-4220, 1980) used with a DHFR selectable marker (e.g., R.J. Kaufman et al. Mol. Biol. 159:601-621, 1982), COS cells, and SP2 cells. Analysis was performed with the GS gene expression system shown in WO 87/04462, WO 89/01036, and EP 338,841.

To produce antibodies, expression vectors encoding anti-ADAM8 or control antibody genes are introduced into mammalian, yeast, or bacterial host cells, and the host cells are cultured for a period of time sufficient for expression of the antibody in the host cells or secretion of the antibody into the culture medium in which the host cells are grown. Antibodies are recovered from the culture medium using standard protein purification methods.

Standard assays to evaluate the binding ability of the antibodies toward any of the various ADAM8 target species ectodomain antigens are known in the art, including for example, ELISAs, western blots, and RIPAs. The binding kinetics (e.g., binding affinity) of the antibodies are assessed by standard assays known in the art, such as by Biacore analysis.

General methodologies for antibody production, including criteria to be considered when choosing an animal or a cell line for the production of antisera, are described in Harlow et al., Antibodies, Cold Spring Harbor Laboratory, pp. 93-117, 1988. For example, an animal of suitable size such as a mouse, pig, goat, rabbit, sheep, primate, monkey, dog, camel, or high value animal, is immunized by administration of an amount of immunogen, such as the intact protein or a portion thereof containing an epitope which is an amino acid sequence from a human ADAM8 ectodomain, effective to produce an immune response. In certain embodiments, the animal is subcutaneously injected in the back with 100 micrograms to 100 milligrams of antigen, dependent on the size of the animal, followed three weeks later with an intraperitoneal injection of 100 micrograms to 100 milligrams of immunogen dependent on the size of the animal with adjuvant, for example Freund's complete adjuvant. Additional i.p. injections every two weeks with adjuvant, for example Freund's incomplete adjuvant, are administered until a suitable titer of antibody in the animal's blood is achieved. Exemplary titers include a titer of at least about 1:5000 or a titer of 1:100,000 or more, i.e., the dilution having a detectable activity. The antibodies are purified, for example, by affinity purification on columns containing the virulence factor or a portion thereof.

The technique of *in vitro* immunization of human lymphocytes is used to generate monoclonal antibodies. Techniques for *in vitro* immunization of human lymphocytes are well known to those skilled in the art (See, e.g., Inai et al. Histochemistry 99(5):335 362, 1993; Mulder et al. Hum. Immunol. 36(3): 186 192, 1993; Harada et al. J. Oral Pathol. Med. 22(4):145 152, 1993; Stauber, et al. J. Immunol. Methods 161(2):157 168, 1993; Venkateswaran, et al. Hybridoma 11(6) 729 739, 1992). These techniques are used to produce antigen-reactive monoclonal antibodies, including antigen-specific IgG, IgA, and IgM monoclonal antibodies.

In various embodiments of the pharmaceutical composition, at least one of the anti-ADAM8 antibody or at least one binding region of the antibody is selected from the group of: a single chain antibody (scFv), a recombinant camelid heavy-chain-only antibody (VHH), a shark heavy-chain-only antibody (VNAR), a microprotein, a darpin, an anticalin, an adnectin, an aptamer, an Fv, a Fab, a Fab', and a F(ab')2.

Peptide libraries used to select an antibody that specifically binds to an ADAM8 protein ectodomain and can be constructed to be displayed on any of various vectors having viable units which are easily manipulated. For example, viable units include any of a phage display library, a *Bacillus* spore display library, an *E. coli* cell display library, and a yeast display library. A vector is used to transfect the viable unit with a gene encoding a protein of interest inserted into a gene expressed on the outside of the unit causing the protein of interest to be displayed on the outside of the phage, yeast cell, or *Bacillus* spore (Marks et al. Journal of Molecular Biology 222(3): 581-597, 5 December 1991; Boder et al. Nature Biotechnology 15, 553-557, 1 June 1997; Isticato et al. J. Bacteriol. 183(21): 6294-6301, 2001). In the Examples herein, the gene codes for an extracellular portion (ectodomain) of ADAM8 protein or for a protein that inhibits an ADAM8 activity.

### Pharmaceutical Compositions

An aspect of the present invention provides pharmaceutical compositions that include at least one of an antibody, antibody chain, or antibody fragment specific for at least part of a target protein which is ADAM8 protein or a fragment thereof, for treating an ADAM8-associated cancer disorder by negatively modulating the amount of ADAM8 expression or activity of the ADAM8 protein or pathway. For example, the modulator is an anti-ADAM8 antibody linked to a toxin. In the application, the pharmaceutical composition is compounded for systemic delivery to a subject, for example the composition is formulated as an injection. The composition in another embodiment is formulated as a systemic formulation for administration to the patient and may be compounded for delivery to affected tissues. In related embodiments, the pharmaceutical composition is formulated sufficiently pure for administration to a human subject, e.g., to the vascular system or endothelial system of a human patient in need of treatment for cancer.

In the application, the compositions herein optionally further include one or more additional therapeutic agents, for example, known or new chemotherapeutic agents. The additional therapeutic agent or agents are selected from the group consisting of growth factors, anti-inflammatory agents, vasopressor agents including but not limited to nitric oxide and calcium channel blockers, collagenase inhibitors, topical steroids, matrix metalloproteinase inhibitors, ascorbates, angiotensin II, angiotensin III, calreticulin, tetracyclines, fibronectin, collagen, thrombospondin, transforming growth factors (TGF), keratinocyte growth factor (KGF), fibroblast growth factor (FGF), insulin-like growth factors (IGFs), IGF binding proteins (IGFBPs), epidermal growth factor (EGF), platelet derived growth factor (PDGF), neu differentiation factor (NDF), hepatocyte growth factor (HGF), vascular endothelial growth factor (VEGF), heparin-binding EGF (HBEGF), thrombospondins, von Willebrand Factor-C, heparin and heparin sulfates, and hyaluronic acid.

In the application, a plurality of therapeutic agents are included in the pharmaceutical composition to treat the same, a concurrent or a related symptom, condition or disease. In various embodiments, the therapeutic agent is a drug that may include without limitation anti-coagulant, anti-tumor, anti-viral, anti-bacterial, anti-mycobacterial, anti-fungal, anti-proliferative or anti-apoptotic agents. In particular, cancer patients frequently present with a bacterial or viral infection due to immune insufficiency, and comorbidity rates are elevated from bacterial pneumonia. Drugs to treat comorbid diagnoses such as bacterial infections that are included in the compositions of the invention are well known in the art. See for example, Goodman & Gilman's The Pharmacological Basis of Therapeutics, 13th Ed., Brenton, L. et al., eds., McGraw-Hill, 2010. For example, in certain embodiments, the therapeutic agent is azithromycin, clarithromycin, erythromycin, doxycycline, gemifloxacin, levofloxacin, moxifloxacin, cefaclor, cefadroxil, cefprozil, cefuroxime, cephalexin, amoxicillin, amoxicillin with clavulanate, ampicillin, piperacillin, ticarcillin with clavulanate, rifamycin, isoniazid, and vancomycin.

An antibody or antibody fragment in various embodiments is an anti-ADAM8 that binds to an ADAM8 ectodomain and is conjugated to an agent so that growth of a target tumor cell is inhibited or the target tumor cell is killed (U.S. patent number 7,226,596). Antibodies are conjugated to anticancer agents, such as cytotoxic agents, chemotherapeutic agents, toxin, or a radioactive isotope, i.e., a radioconjugate to form an immunoconjugate (U.S. patent application publication number 2008/0175842). A variety of radionuclides are covalently bound or complexed as chelates to form radioconjugated antibodies (U.S. patent number 7,226,596), e.g., ²¹²Bi, ¹³¹I, ¹³¹In, ⁹⁰Y and ¹⁸⁶Re. (Ibid.). Toxins for immunoconjugates include diphtheria A chain, nonbinding active fragments of diphtheria toxin, cholera toxin, botulinus toxin, exotoxin A chain, ricin A chain, abrin A chain, modeccin A chain, alpha-sarcin, *Aleurites fordii* proteins, dianthin proteins, *Phytolaca americana* proteins (PAPI, PAPII, and PAP-S), *momordica charantia* inhibitor, curcin, crotin, *Sapaonaria officinalis* inhibitor, gelonin, saporin, mitogellin, restrictocin, phenomycin, enomycin and the tricothecenes. (U.S. patent application publication number 2008/0175842). Small molecule toxins for conjugating to anti-ADAM8 antibodies include calicheamicins (U.S. patent number 5,053,394), maytansinoids (U.S. patent number 5,208,020), palytoxin, and CC1065 (U.S. patent number 7,226,596).

Bifunctional protein coupling agents are used to make antibody/cytotoxic agent immunoconjugates, e.g., N-succinimidyl-3-(2-pyridyldithiol) propionate (SPDP), iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl)hexanediamine), bis-diazonium derivatives (such as bis-(p-diazoniumbenzoyl)-ethylenediamine), diisocyanates (such as tolyene 2,6-diisocyanate), and bis-active fluorine compounds, such as 1,5-difluoro-2,4-dinitrobenzene (U.S. patent application publication number 2008/0175842). For example, preparation of a ricin immunotoxin is described in Vitetta et al. Science 238:1098 (1987) (Ibid.).

Two conjugated antibodies have been approved for use in the clinic. For example, T-DM1 (trastuzumab emtansine, the first antibody-drug conjugate) was approved February 2014 for patients with metastatic breast cancer that overexpresses HER2. T-DM1 is an immunoconjugate of trastuzumab with DM1, a chemotherapy drug.

As used herein, the term "pharmaceutically acceptable carrier" includes any and all solvents, diluents, or other liquid vehicle, dispersion or suspension aids, surface active agents, isotonic agents, thickening or emulsifying agents, preservatives, solid binders, lubricants, and the like, as suited to the particular dosage form desired. Remington's Pharmaceutical Sciences, 18th Ed.; Gennaro, Mack Publishing, Easton, PA (1995) provides various carriers used in formulating pharmaceutical compositions and known techniques for the preparation thereof. Examples of materials which serve as pharmaceutically acceptable carriers include, but are not limited to, sugars such as glucose and sucrose; excipients such as cocoa butter and suppository waxes; oils such as peanut oil, cottonseed oil, safflower oil, sesame oil, olive oil, corn oil, and soybean oil; glycols such a propylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol; and phosphate buffer solutions, as well as other non-toxic compatible lubricants such as sodium lauryl sulfate and magnesium stearate, as well as coloring agents, releasing agents, coating agents, preservatives and antioxidants can also be present in the composition, the choice of agents and non-irritating concentrations to be determined according to the judgment of the formulator.

### Therapeutically Effective Dose

The claimed use in treatment of breast cancer provided herein involves contacting cancer or pre-cancerous or tumor cells or tissues with a pharmaceutical composition that modulates or inhibits ADAM8 amount or activity, for example, administering to a subject having the cancer or cells or tissue a therapeutically effective amount of a pharmaceutical composition having as an active agent at least one of an antibody specific for ADAM8, to the subject in need thereof, in such amounts and for such time as is necessary to achieve the desired result which is reduction of or preventing indicia of the cancer-related condition.

The compositions, may be administered using any amount and by any route of administration effective for treating the cancer-related disorder. Thus, the expression "amount effective for treating a cancer-related disease or condition", as used herein, refers to a sufficient amount of the composition to beneficially prevent or ameliorate the symptoms of the disease or condition.

The exact dosage is chosen by the individual physician in view of the patient to be treated. Dosage and administration are adjusted to provide sufficient levels of the active agent(s) or to maintain the desired effect. Additional factors which may be taken into account include the severity of the disease state, *e.g.,* liver function, cancer progression, and/or intermediate or advanced stage of macular degeneration; age, weight and gender of the patient; diet, time and frequency of administration; route of administration; drug combinations; reaction sensitivities; and tolerance/response to therapy. Long acting pharmaceutical compositions might be administered hourly, twice hourly, every three to four hours, daily, twice daily, every three to four days, every week, or once every two weeks depending on half-life and clearance rate of the particular composition.

The active agents of the anti-ADAM8 compositions of the invention are preferably formulated in dosage unit form for ease of administration and uniformity of dosage. The expression "dosage unit form" as used herein refers to a physically discrete unit of active agent appropriate for the patient to be treated. The total daily usage of the compositions of the present invention will be decided by the attending physician within the scope of sound medical judgment. For any active agent, the therapeutically effective dose can be estimated initially either in cell culture assays or in animal models, as provided herein, usually mice, but also potentially from pigs, goats, rabbits, sheep, primates, monkeys, dogs, camels, or high value animals. The cell-based, animal, and *in vivo* models provided herein are also used to achieve a desirable concentration and total dosing range and route of administration. Such information can then be used to determine useful doses and routes for administration in humans.

A therapeutically effective dose refers to that amount of active agent that ameliorates the symptoms or condition or prevents progression of the disease or condition. Therapeutic efficacy and toxicity of active agents are determined by standard pharmaceutical procedures in cell cultures or experimental animals, e.g., ED₅₀ (the dose is therapeutically effective in 50% of the population) and LD₅₀ (the dose is lethal to 50% of the population). The dose ratio of toxic to therapeutic effects is the therapeutic index, which can be expressed as the ratio, LD₅₀/ED₅₀. Pharmaceutical compositions which exhibit large therapeutic indices are preferred. The data obtained from cell culture assays and animal studies are used in formulating a range of dosage for human use.

The daily dosage of the products may be varied over a wide range, such as from 0.1 mg to 500 mg per adult human per day. For intravenous or local intra-tumoral administration, the compositions are provided for example in the form of a solution containing at least about 0.001 mg, about 0.01 mg, about 0.05 mg, about 0.1 mg, about 0.5 mg, about 1 mg, about 5 mg, about 10 mg, about 25 mg, about 50 mg, about 75 mg, about 100 mg, about 250 mg, or about 500 mg of the active ingredient for the symptomatic adjustment of the dosage to the patient to be treated.

A unit dose typically contains from about 0.001 mg to about 500 mg of the active ingredient, preferably from about 0.1 mg to about 100 mg of active ingredient, more preferably from about 1.0 mg to about 10 mg of active ingredient. An effective amount of the drug is ordinarily supplied at a dosage level of from about 0.01 mg/kg to about 200 mg/kg of body weight. For example, the range is from about 0.01 to 100 mg/kg of body weight per day, or from about 0.5mg/kg to 50 mg/kg of body weight per day, or from about 1.0 mg/kg to about 25 mg/kg of body weight per day. The compositions may be administered on a regimen of, for example, one to four or more times per day or per week. A unit dose may be divided for example, administered in two or more divided doses.

### Administration of Pharmaceutical Compositions

As formulated with an appropriate pharmaceutically acceptable carrier in a desired dosage, the anti-ADAM8 antibody pharmaceutical composition or ADAM8 expression modulator provided herein is administered to humans and other mammals for example topically for skin tumors (such as by powders, ointments, creams, or drops), orally for cancers of the mouth and tongue, rectally, mucosally, sublingually, parenterally, intracisternally, intravaginally, intraperitoneally, intravenously, subcutaneously, bucally, sublingually, ocularly, or intranasally, depending on preventive or therapeutic objectives and the severity and nature of the cancer-related disorder or condition.

Injections of anti-ADAM8 antibodies include intravenous injection or intra-ocular injection into the tumor directly, for example, for esophageal, breast, brain, head and neck, and prostate tumors.

Liquid dosage forms for example for intravenous, ocular, mucosal, or other administration include, but are not limited to, pharmaceutically acceptable emulsions, microemulsions, solutions, suspensions, syrups and elixirs. In addition to at least one active agent, the liquid dosage forms may contain inert diluents commonly used in the art such as, for example, water or other solvents; solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols, and fatty acid esters of sorbitan; and mixtures thereof. Besides inert diluents, the ocular, oral, or other systemically-delivered compositions can also include adjuvants such as wetting agents, and emulsifying and suspending agents.

Dosage forms for topical or transdermal administration of an inventive pharmaceutical composition include ointments, pastes, creams, lotions, gels, powders, solutions, sprays, inhalants, or patches. The active agent is admixed under sterile conditions with a pharmaceutically acceptable carrier and any needed preservatives or buffers as may be required. For example, ocular or cutaneous routes of administration are achieved with aqueous drops, a mist, an emulsion, or a cream. Administration may be therapeutic or prophylactic. The invention includes implantation devices, surgical devices, or products which contain disclosed compositions (e.g., gauze bandages or strips), and methods of making or using such devices or products. These devices may be coated with, impregnated with, bonded to or otherwise treated with a composition as described herein.

Transdermal patches have the added advantage of providing controlled delivery of the active ingredients to the eye and body. Such dosage forms can be made by dissolving or dispensing the compound in the proper medium. Absorption enhancers can also be used to increase the flux of the compound across the skin. The rate can be controlled by either providing a rate controlling membrane or by dispersing the compound in a polymer matrix or gel.

Injectable preparations of anti-ADAM8 antibody, for example, sterile injectable aqueous or oleaginous suspensions may be formulated according to the known art using suitable dispersing or wetting agents and suspending agents. The sterile injectable preparation may also be a sterile injectable solution, suspension or emulsion in a nontoxic parenterally acceptable diluent or solvent, for example, as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono-glycerides or diglycerides. In addition, fatty acids such as oleic acid are used in the preparation of injectables. The injectable formulations can be sterilized, for example, by filtration through a bacterial-retaining filter, by irradiation, or by incorporating sterilizing agents in the form of sterile solid compositions which can be dissolved or dispersed in sterile water or other sterile injectable medium prior to use. In order to prolong the effect of an active agent, slowing absorption of the agent from subcutaneous or intratumoral injection is often desirable. Delayed absorption of a parenterally administered active agent may be accomplished by dissolving or suspending the agent in an oil vehicle. Injectable depot forms are made by forming microencapsule matrices of the agent in biodegradable polymers such as polylactide-polyglycolide. Depending upon the ratio of active agent to polymer and the nature of the particular polymer employed, the rate of active agent release can be controlled. Examples of other biodegradable polymers include poly (orthoesters) and poly(anhydrides). Depot injectable formulations are also prepared by entrapping the agent in liposomes or microemulsions which are compatible with body tissues.

The invention provides an ADAM8 ectodomain-binding antibody or the antigen-binding fragment thereof for use in the treatment of breast cancer or breast cancer associated metastasis characterized in that the antibody or the antigen-binding fragment thereof binds to SEQ ID No 9 and that the antibody or the antigen-binding fragment thereof inhibits the activity of the metalloproteinase domain and disintegrin domain of ADAM8.

The claimed use provides an anti-ADAM8 antibody that binds to the ADAM8 ectodomain, produced having specific affinity to an antigen having an amino acid sequence of at least one ADAM8 protein ectodomain selected from a disintegrin domain (DI) and a metalloproteinase domain (MP). The immunizing antigen is at least four to seven amino acids in length, and may be as long as the entire ectodomain having length of several hundred amino acids.

In the embodiment of breast cancer, the use in the treatment includes breast cancer that is triple-negative or that is estrogen receptor negative. The claimed use includes the cancer which is a primary tumor, for example, the tumor is non-metastatic. Alternatively, the claimed use includes a cancer that is metastatic. In said use, the subject is a human; alternatively the subject is a high value animal or is a research animal.

The antibody in the application is obtained by immunizing or selecting from a yeast or phage or other type of display library with a peptide of an ADAM8 protein having an amino acid sequence selected from sequences as shown in any of: SEQ ID NO: 4; SEQ ID NO: 5; SEQ ID NO: 6; SEQ ID NO: 7; and SEQ ID NO: 9 as a target. For example, the antibody is obtained by immunizing with a peptide including an amino acid sequence within residues 158 to 653 corresponding to the ectodomain of ADAM8, or selecting for a member of the library that binds to the ectodomain or ectodomain fragment.

The application further involves analyzing a decrease in at least one symptom of cancer or metastasis progression. Alternatively, the symptom is at least one selected from: decreased size of primary tumor; cessation of growth of primary tumor; decreased number of metastases; decreased size of metastases; absence of new metastases; decreased number of circulating tumor cells in blood; decreased vascularization in tissue at the periphery of or within the tumor or metastases; decreased amount of ADAM8 in body fluids; decreased amount of VEGF-A in body fluids; and, weight loss of the subject.

The application provides a method of diagnosing or prognosing a presence of a cancer disease condition in a patient and selecting a course of treatment for the patient, the method including: analyzing amount of expression of ADAM8 protein in the patient or patient tumor; measuring the amount in a normal subject or in an unaffected tissue of the cancer patient, such that an increase in expression in the cancer of the patient in comparison to the normal subject or the normal tissue of the patient is an indicium of the cancer; and, prognosing a course of treatment for the patient, such that the indicium of ADAM8-related cancer guides selecting at least one therapeutic agent that inhibits or reduces expression or activity of ADAM8.

An embodiment of the claimed use further involves administering to the patient a therapeutically effective amount of an antibody or antibody fragment that binds to an extracellular portion (ectodomain) of ADAM8 protein and inhibits the activity of ADAM8.

The activity of ADAM8 is at least one of: ADAM8 metalloproteinase enzyme activity; release of VEGF-A; promotion of angiogenesis; extravasation or intravasation of cancer cells through vascular endothelium; and β1-integrin activation.

The application provides also a method of screening a library of compounds to identify a candidate therapeutic agent to treat a cancer, the method including:
contacting a first ADAM8 sample with at least one compound, and contacting a second ADAM8 sample with a control lacking the compound and otherwise identical; analyzing amounts of ADAM8 or an ADAM8 activity in the first and second samples, such that a reduced amount or reduced activity in the first sample compared to the second sample identifies the compound as the candidate therapeutic agent; testing the compound for at least one of chemical structural and biological functional properties; and, testing the compound for ADAM8 specificity by determining if there is cross-reactivity with closely-related ADAM family members: ADAM9, ADAM10, ADAM12, ADAM15, ADAM17, ADAM19, and ADAM33. The specific compound inhibits ADAM8 and does not inhibit closely related ADAM family members.

### Examples

### Example 1. Cells and Culture Conditions

Human TNBC cell lines (MDA-MB-231, Hs578T, MDA-MB-468, BT549), non-tumoral mammary epithelial cell line MCF-10A, and umbilical vein endothelial cell line HUVEC were purchased from the American Type Culture Collection (ATCC) and maintained in medium recommended by ATCC. Inflammatory breast cancer cell line SUM149 was maintained as described in Forozan et al. Br J Cancer 81(8): 1328-1334, 1999. MDA-MB-231-derived LM1 and LM2 cells were isolated *in vivo* because of the cells' ability to metastasize to the lung (Minn et al. Nature 436:518-524, 2005). Stable clones of *ADAM8* shRNA (shA8) and *Control* shRNA (shCtrl) MDA-MB-231 cells, expressing GFP, were established using transfection of either *ADAM8* shRNA or scramble control vector (Origene), respectively, using LTX Lipofectamine (Invitrogen), selection with 1 µg/ml puromycin (Sigma), and limiting dilution. MDA-MB-231 clones were authenticated using short tandem repeat analysis (Genetica DNA Laboratories), which demonstrated 100% identity with the MDA-MB-231 cell line of ATCC. For culture in 3D suspension conditions, single-cell suspensions in complete media were plated at 1.25 x 10⁴ cells per well in ultra low-attachment 6-well plates (Costar) and incubated for 48 h. For culture in hypoxic conditions, cells were incubated in 1% oxygen for 6 or 24 h in 6-well plates.

### Example 2. siRNA Knockdown Analyses

Transient RNAi-mediated ADAM8 knockdown was performed with the following short interfering RNAs (siRNAs; QIAGEN):
siADAM8 (siA8)#1 (Hs_ADAM8_6): 5'-CGGCACCTGCATGACAACGTA-3' (SEQ ID NO: 1) and
siA8#2 (Hs_ADAM8_7): 5'-CTGCGCGAAGCTGCTGACTGA-3' (SEQ ID NO: 2).

AllStar negative control siRNA (QIAGEN) was used in each example as a non-silencing control siRNA (siCtrl). siRNAs (10 nM) were introduced into cells using Lipofectamine RNAi Max Transfection Reagent (Invitrogen) by reverse transfection according to the manufacturer's protocol. Transfected cells were used 24 h later in functional assays.

The target sequence of the *ADAM8* shRNA used in this study was GCGGCACCTGCATGACAACGTACAGCTCA (SEQ ID NO: 8).

### Example 3. Western Blotting

WCEs from cells in culture or frozen homogenized tumor tissue were prepared using radio-immunoprecipitation assay (RIPA) buffer supplemented with protease and phosphatase inhibitors (Pierce), and further supplemented with EDTA and phenanthroline to inhibit the autocatalytic activity of ADAM8. Samples (25 µg) were analyzed by immunoblotting as described in Romagnoli et al. Cancer Res 72:6268-6278, 2012. Antibodies specific for ADAM8 were purchased from Millipore (AB19017) and LSBio (B4068). Antibody specific for VEGF-A was purchased from R&D Systems (AF293NA). Antibodies specific for β-Actin (AC-15) and β-tubulin (TUB 2.1) were purchased from Sigma. HIF-1α antibody (NB100-105) was purchased from Novus Biologicals. Antibodies against HA-tag (sc-805) and Lamin B (sc-6217) were purchased from Santa Cruz Biotechnology. For western blot analysis of released VEGF-A, serum-free medium incubated with cells for 16 h was collected, centrifuged at 4,000 rpm for 10 min to remove cell debris and was concentrated using centrifugal 10K filters (Amicon Ultra-15). Volumes of the conditioned medium corresponding to 2.5 x 10⁵ cells were analyzed by immunoblotting using a VEGF-A antibody (R&D Systems, MAB293).

### Example 4. ATP Assay

As a measure of cellular metabolism and therefore cell growth, ATP levels were assessed in 3 x 10³ MDA-MB-231 cells using an ATPlite luminescence ATP detection assay system (Perkin Elmer), as described in Mineva et al. J Cell Physiol; 220(3):593-9, 2009. Average ATP levels for triplicate samples are presented relative to control condition set to 1 (Mean ± S.D. from three independent examples).

### Example 5. Soft Agar and Matrigel Outgrowth Assays

For soft agar assays, 5 x 10⁴ cells in a mix of 0.4% Bacto Agar (BD Biosciences) in complete media were plated in triplicate on 6-well dishes pre-coated with a 1:1 mix of 2x DMEM medium supplemented with 10% FBS and 1.6% Bacto Agar. Cells were fed three times per week with complete medium. After 8-12 days, cells were stained overnight with 0.2 mg/ml iodonitrotetrazolium chloride (Sigma) and photographed at 10x magnification. Colonies with diameters of approximately 20 µm or greater were counted using ImageJ software (NIH). Matrigel outgrowth assays were carried out as in Wang et al. Nat Cell Biol 9:470-478, 2007, using 5 x 10³ cells plated in duplicate on 24-well plates. Cultures were incubated for 4-7 days and photographed at 20x magnification.

### Example 6. Migration/Invasion and Transendothelial Migration Assays

Migration and invasion assays were performed in triplicate using polycarbonate filter Transwells (Costar) with 8-µm-diameter pores, without precoating or precoated with growth factor-reduced Matrigel (BD Biosciences, 356231), respectively. Transendothelial migration assays were performed as described herein with Transwells precoated with HUVECs that had been allowed to grow until a confluent monolayer was achieved. Suspensions of 1 x 10⁵ tumor cells were layered in the upper compartments of the Transwell and incubated at 37°C. After 24 h, number of cells that migrated or invaded to the lower side of the filter were evaluated by crystal violet staining and OD₅₇₀ₙₘ determination. The control condition (siCtrl, shCtrl, or isotype antibody control) was set to 100% and the mean ± S.D. from three independent replicated assays is given.

### Example 7. Tube Formation Assay

Tube formation assays were performed as described (Kim et al. Mol Cancer Res 5:321-329, 2007). Briefly, 2 x 10⁴ HUVEC cells were plated, in triplicate, on glass chambers coated with growth factor reduced Matrigel. Cells were incubated in 200 µl EBM-2 Basal Medium supplemented with 1% FBS and concentrated conditioned medium from shCtrl and shA8 MDA-MB-231 clones (volume corresponding to 5 x 10⁵ tumor cells) or conditioned media in the absence of tumor cells. Following incubation for 13 h, three random fields per well were photographed (40x magnification). Incomplete networks were excluded and numbers of closed networks of vessel-like tubes counted in three fields (n = 9). The average percentage relative to control samples is presented ± S.D. from a representative of three independent replicated assays.

### Example 8. Endothelial Cell Adhesion Assay

Endothelial cell adhesion assays were performed as described in Price et al. Br J Cancer 74:1762-1766, 1996. Briefly, 1 x 10⁵ HUVEC cells were plated, in duplicate, in 48-well plates. After 24 h, 5 x 10⁴ shCtrl or shA8 MDA-MB-231 cells stained with NucBlue (Invitrogen) were incubated for 15 min at 37°C in 300 µl of EBM-2 Basal Medium supplemented with 1% FBS on top of the confluent HUVEC monolayer or in empty wells (without HUVECs). Unattached MDA-MB-231 cells were washed three times with PBS and attached NucBlue-positive cells were counted in three random fields per well (n = 6). The average percentage relative to control samples is presented ± S.D. from three independent examples. For inhibition examples with antibodies, tumor cells and/or HUVECs were pre-treated with 10 µg/ml of β1-integrin antibody (BD Biosciences, 552828), 20 µg/ml of antibody specific for an ADAM8 ectodomain (R&D Systems, MAB10311 and Mab1031), or control isotypes (rat or mouse, respectively) for 30 min at room temperature and then washed twice with phosphate buffered saline (PBS) before being used in the endothelial cell adhesion assay.

### Example 9. Human Angiogenesis Array

Secreted levels of 55 angiogenesis related proteins were analyzed in the conditioned medium from shControl (shCtrl-3 and -5) and shADAM8 (shA8-17 and -20) MDA-MB-231 clones using the Proteome Profiler™ Human Angiogenesis antibody array (R&D Systems, ARY007). Cells incubated with 2% fetal bovine serum (FBS) conditioned medium for 24 h were centrifuged at 4,000 rpm for 10 min to remove cell debris and a volume of medium corresponding to 2.5 x 10⁵ cells was analyzed by the antibody array according to the manufacturer's protocol. The signal intensity of the detected proteins (two spots for each protein) was measured using ImageJ, the background signal subtracted, and the average intensity (mean ± S.D) for the two clones was calculated.

### Example 10. Mammary Fat Pad (MFP) Mouse Model

Six-week-old female nonobese diabetic/severe combined immunodeficient (NOD/SCID) mice (Jackson Laboratory) were implanted with 2.5 x 10⁶ cells in 50 µL 50% Matrigel (BD Biosciences, CB-40230A) solution (1:1 dilution of Matrigel with DMEM medium) in the fourth inguinal mammary fat pad. Primary tumor growth was monitored by caliper measurement twice a week. Tumor volumes were calculated as (length × width²)/2. Mice were sacrificed after 3-4 weeks when tumors derived from shCtrl MDA-MB-231 cells had reached a volume of about 1 cm³. Mice were photographed, tumors were dissected, photographed, weighed and fixed in 10% Neutral Buffered Formalin for histological and IHC analyses. For metalloproteinase/disintegrin antibody treatment examples, control isotype IgG2B (n = 8) or antibody for ADAM8 (anti-A8 mAb; n = 9; Mab004 and Mab1031 respectively, R&D Systems) was injected i.p. twice weekly at the dosage of 0.5 mg/kg from the day of shCtrl-3 cell implantation in the MFP.

For the cysteine-rich/EGF-like antibody treatment examples, control isotype IgG1 (n=7) or anti-A8 Ab (n=7; Mab002 and Mab10311, R&D Systems) was injected i.p. twice weekly at 1.5 mg/kg from the day of shCtrl-3 cell implantation in the MFP.

Alternatively, treatment with doses of 1.5 mg/kg was performed using a dosage regiment of administering treatment after the primary tumors became palpable and continuing with an injection on the day of resection when the tumors reached a volume of about 0.15 cm³ and twice weekly for the following five weeks. For metastasis detection, brains and lungs were removed by dissection, stored in cold PBS and examined for GFP expression using a fluorescent dissecting microscope (Leica MZ FLIII).

### Example 11. Cardiac Colonization Mouse Model

Six-week-old female nonobese diabetic/severe combined immunodeficient (NOD/SCID) mice (Jackson Laboratory) were implanted with 0.5 x 10⁶ cells in 100 µL PBS using an ultrasound guided injection with a 27½-gauge needle into the left ventricle. Immediately following cardiac injection, systemic vascular dissemination of the tumor cells was analyzed by detection of Luciferase activity using the Xenogen Biophotonic Imaging system. Total tumor burden was monitored weekly as described herein. Mice were sacrificed three weeks after initial cell injection. Organs and bones were harvested and specific Luciferase activity detected as described herein.

### Example 12. Detection of CTCs

Blood was collected at sacrifice either by cardiac puncture (about 500 µl; day 33), or by drawing from mice using the submandibular blood collection method (about 100 µl) at time points 7, 14 and 21 days after inoculation of tumor cells into the MFP. To evaluate level of autofluorescence, blood was collected also from control NOD/SCID mice that were not injected with any tumor cells. Blood samples were collected in heparin (100 USP units/sample), and were diluted and processed the same day for detection of GFP-positive CTCs using a confocal flow cytometry system at a flow rate of 4.5 µl per min, as described in Greiner et al. Cytometry A 79:874-883, 2011. Enumerated cells were identified with an algorithm as described in Hwu et al. J Biomed Opt 16:040501, 2011 based on the combined detection of peaks appearing simultaneously in a fluorescence channel optimized for GFP detection and in at least one of the channels detecting scattering at either 405 or 488 nm.

Diluted whole blood samples were flowed in microfluidic devices, each having five single 50 µm (width) by 70 µm (height) and 10 mm (length) channels. To flow cells in a microfluidic channel for light scattering and fluorescence emission measurements, the outlet tubing was placed into eppendorf tubes and the cell suspension was drawn by a syringe pump (Harvard Apparatus) at a flowrate of 6 µl min⁻¹. Operating at a constant pressure, a 6 µl min⁻¹ set flowrate calibrated for water yielded an actual value of 4.5 µl min⁻¹ due to the increased viscosity of blood. Channels were pre-wetted by flowing PBS, and the blood sample flow was allowed to stabilize prior to data acquisition. Of the initial approximately100 µl of whole blood collected, 34±17, 55±17, and 57±9 µl, respectively, were analyzed for non-tumor bearing, *shControl,* and *shADAM8* mice, respectively. At the final 33 day time point, 100 µl of blood was analyzed.

Counts positive for GFP fluorescence were detected in the 500 to 590 nm "green" channel following excitation with 488 nm light and were time-correlated with 405 and 488 nm scattering values of sufficient intensity. The incident 633 nm light interrogated cells for red autofluorescence, which was detected in the 650-690 nm channel and analyzed separately from green correlated counts. Within the volume analyzed, leukocytes, and CTCs constituted approximately 0.5% of the acquired backscattered and fluorescent light data stream. Using a 25 kHz sampling frequency, the lower 99.5% of the intensity values defined the mean and standard deviation of the background signal. Individual peaks were demarcated by 6 to 7 data points and the maximum value defined the intensity used to determine whether the count contained sufficiently intense scattering and fluorescence values for CTC identification. Within a Matlab program, the intensity threshold was defined based on the number of standard deviations above the mean of the background signal. The number of standard deviations was observed to be, respectively, 4, 4, 7, and 5 for the 405, 488, 650-690, and 500-590 nm channels, respectively. Of the non-tumor bearing samples assayed, a concentration of mean ± S.D. µl⁻¹ green autofluorescent cells were detected and subtracted from the detected counts in tumor-bearing samples, yielding the reported CTC concentration values.

### Example 13. ADAM8 Metalloproteinase Activity Assay

HEK-293 cells were plated at 5 x 10⁵ cells/ml in P60 dishes. After 24 h, cultures were co-transfected with 1 µg of plasmid DNA encoding C-terminal HA-tagged CD23 (CD23 membrane isoform b, purchased from Addgene), and 1 µg vector DNA expressing either ADAM8 full-length or remnant form, or empty vector (EV) pCDNA3.1 Version B DNA. The medium was replaced after 6 h with culture medium without FBS. After 16 h, the serum-free media was harvested and centrifuged to remove cell debris, and cells were trypsinized and counted. The supernatants were concentrated using Amicon-ultra 4 centrifugal filter units (Millipore), and volumes corresponding to equal cell numbers were assessed for cleaved CD23 using Western blotting. WCEs from lysed cells were assessed for ADAM8 levels.

### Example 14. Immunohistochemistry of Patient Samples

Paraffin-embedded tissues from Formalin-fixed tumors were selected (see Table 1 and Figures 1D-1F) and were analyzed for ADAM8 protein expression by immmunohistochemistry.

**Table 1. Clinical characteristics of the primary and metastatic breast tumors assayed for ADAM8 protein expression by immunohistochemistry, including 37 Ductal Carcinoma In situ (DCIS), 50 Triple-Negative Breast Cancers (TNBCs) and 56 metastases. N(%) is shown in parentheses.**

| Tumor characteristic | DCIS | Triple-negative | Metastasis |
|---|---|---|---|
| Age | | | |
| ≤ 50 yrs | 12 (32.4) | 13 (26.0) | 0 |
| > 50 yrs | 25 (67.6) | 37 (74.0) | 0 |
| Unknown | 0 | 0 | 56 (100.0) |

| Grade | | | |
|---|---|---|---|
| 1 & 2 | 0 | 14 (28.0) | 4 (7.1) |
| 3 | 0 | 35 (70.0) | 9 (16.1) |
| Unknown | 37 (100.0) | 1 (2.0) | 43 (76.8) |

| ER Status | | | |
|---|---|---|---|
| Negative | 0 | 50 (100.0) | 6 (10.7) |
| Positive | 0 | 0 | 7 (12.5) |
| Unknown | 37 (100.0) | 0 | 43 (76.8) |

| HER2 Status | | | |
|---|---|---|---|
| Negative | 0 | 50 (100.0) | 8 (14.3) |
| Positive | 0 | 0 | 5 (8.9) |
| Unknown | 37 (100.0) | 0 | 43 (76.8) |

| Nodal Status | | | |
|---|---|---|---|
| Negative | 0 | 33 (66.0) | 6 (10.7) |
| Positive | 0 | 16 (32.0) | 5 (8.9) |
| Unknown | 37 (100.0) | 1 (2.0) | 45 (80.4) |

| Histology | | | |
|---|---|---|---|
| Ductal | 37 (100.0) | 44 (88.0) | 12 (21.4) |
| Lobular | 0 | 3 (6.0) | 1 (1.8) |
| Others | 0 | 3 (6.0) | 0 |
| Unknown | 0 | 0 | 43 (76.8) |

Specimens were cut in 3-µm-thick sections and stained with H&E. Alternatively, sections were processed for immunohistochemistry. Sections were deparaffinized in xylene and rehydrated through an alcohol gradient, and endogenous peroxidase activity was blocked with 3% hydrogen peroxide. Samples were steamed before incubation for antigen retrieval with citrate buffer (pH 6.0). Slides were incubated with a primary antibody targeting ADAM8 (1:150, B4068, LSBio) on an automated immunostainer using a labelled streptavidin-biotin method (Dako) followed by 3,3'-diaminobenzidine chromogen detection. Immunostained slides were counterstained with hematoxylin. Negative controls were included in each run by omitting the primary antibody. ADAM8 immunoreactivity in carcinomatous cells was scored by a pathologist using a five-tiered semiquantitative system (0: no staining; 1+: focal weak staining <10%; 2+: 10-25%; 3+: 25-50%; 4+: >50%). Non-carcinomatous cells were excluded from scoring.

### Example 15. Immunohistochemistry of Mouse Samples

Primary antibodies targeting ADAM8 (1:150, B4068, LSBio), CD31 (1:50, ab28364, Abcam) and the active conformation of β1-integrin (1:100, B2861, LSBio) were used in the same protocol described as herein for patient specimens. For evaluation of vessel density, two slides/tumor were cut 200 µm apart and analyzed by immunostaining for CD31. Slides were examined at low magnification and three areas with the greatest numbers of vessels (hot spots) at the tumor periphery were identified for each tumor slice. These were photographed at 40x magnification, the numbers of vessels/hotspot counted and the average value of vessel density/tumor plotted (n = 6/tumor).

### Example 16. Gene Expression and Statistical Analysis

Cancer datasets were downloaded from ONCOMINE™ (Compendia Bioscience, www.oncomine.com), as described in Romagnoli et al., 2012. The rank for *ADAM8* across the 14 analyses and its P-value for the comparison of *ADAM8* expression between breast carcinoma and normal breast, were obtained directly from the ONCOMINE™ website. A comprehensive gene expression microarray data set of 295 primary breast tumors (obtained from Rosetta Inpharmatics) prepared by van de Vijver and colleagues (van de Vijver et al. N Engl J Med 347:1999-2009, 2002), was used to compare *ADAM8* mRNA levels across the different molecular breast cancer subtypes and to assess correlation of this protein with clinicopathological variables and patient outcome. Univariate relationships between *ADAM8* mRNA log ratio data and patient outcome were assessed using the Log-rank test (RR, CI). A RR (relative risk) of 1 indicates that the risk of relapse or mortality is the same in both groups, while a RR of more than 1 indicates an increased risk. Confidence intervals (CIs) were calculated for the RR, which was then tested for statistical significance using the Wald test. Data were assigned arbitrary cut-off points. Data shown are representative of results obtained using the 75^{th} percentile. Results are presented using Kaplan-Meier curves. *P*-values less than 0.05 (P < 0.05) were regarded as being statistically significant. Data were analyzed using PASW statistics version 18.0 (SPSS Inc.). The Pearson's pairwise correlation plot showing the correlation between *ADAM8* and *CD31* (*PECAM1*) expression was obtained using breast tumor microarray datasets from the bc-GenExMiner Database (http://bcgenex.centregauducheau.fr). All other analyses were performed using a two-tailed Student's *t*-test for samples with equal variance, in which P-values less than 0.05 were considered statistically significant.

Analysis of the ONCOMINE™™ database was used to compare the copy number of ADAM8 DNA in breast cancer samples with that of normal breast tissue. No copy number gain was found among samples from invasive ductal and lobular carcinoma (n = 14), invasive ductal (n = 647), invasive lobular (n = 71), mixed lobular and ductal (n = 9), or mucinous (n = 9) cancerous tissues as compared to normal breast tissue (n = 111). In fact, a decrease in copy number was observed for invasive ductal tissue compared to normal breast tissue, and for mucinous compared to normal breast tissue, as is typical of deletions that arise during cancer.

### Example 17. High ADAM8 Expression in Human Breast Tumors Correlates with Poor Prognosis

The ONCOMINE™ microarray database was used to assess *ADAM8* mRNA levels in breast cancer. *ADAM8* levels were observed significantly overexpressed in invasive breast carcinoma samples from different origins (ductal, lobular, mixed), in comparison to normal breast tissues (Figure 1A). In addition, for tumor samples were stratified according to ERα status, *ADAM8* levels were observed to be significantly higher in ERα- (Estrogen receptor negative) breast carcinomas (Figure 1B), which are known for a poorer prognosis compared to ERα+ ones. Importantly, *ADAM8* was identified as one of the more highly expressed genes in human breast tumors in compared to normal breast tissue (Figure 1C).

IHC analysis was performed to assess ADAM8 expression directly in breast tumors (Figure 1D-1E). ADAM8 staining was localized to the cytoplasm and plasma membrane of cancer cells, and was observed abundantly in 34.0% (17/50) of TNBC (Figure 1D-E). Interestingly, at primary tumor sites, ADAM8 expression was detected at the leading front of microinvasive areas (Figure 1E, bottom). In contrast, ADAM8 was not detectable in adjacent normal mammary tissue of TNBC (Figure 1E, top). In addition, only 13.5% (5/37) of ductal carcinoma *in situ* (DCIS) tumors, which are defined by the lack of local invasion out of the mammary ducts, were positive for ADAM8 staining (Figure 1D).

IHC analysis of 56 human breast cancer-derived metastases revealed that 48.2% (27/56) were positive for ADAM8 expression (Figure 1D and 1F), including the majority of brain metastases (63%), and significant percentages of lymph nodes (44.5%), liver (29.4%) and other organ metastases (33.3%, 1/3). Consistently, using bc-GenExMiner database, high *ADAM8* levels correlated with metastasis relapse, as indicated by the Kaplan-Meier curve resulting from data containing more than 2,000 breast cancer samples from patients and 600 events (Figure 1G).

These findings demonstrate that ADAM8 is aberrantly expressed in a large number of breast cancers, in particular in patients with TNBC, and expression of ADAM8 is associated with a worse prognosis. Thus, a role of ADAM8 was observed in TNBC, cancers which are characterized by high rates of recurrence and dissemination to distant organs and have fewer effective targeted therapies (Boyle, 2012).

### Example 18. ADAM8 Expression in TNBC Cells is Increased in 3D Suspension Cultures

ADAM8 is synthesized as a 120 kD proform, which multimerizes and autocatalytically clips off its prodomain, leaving a proteolytically active 90 kD membrane-anchored metalloproteinase, which can be further processed to a 60 kD remnant form (Figure 2A; Koller et al. Curr Pharm Des 15:2272-2281, 2009).

Using western blot analysis, high ADAM8 proform expression was found in all TNBC cell lines tested compared to the non-tumoral mammary epithelial cell line MCF-10A when grown in adherent conditions (Figure 2B). Processed active and remnant ADAM8 forms were prominent in MDA-MB-231 and SUM149 cells in 2D-culture. Growth of MDA-MB-231 and Hs578T cells under low attachment conditions, which selects for a more aggressive phenotype, was used to further study cells with or without evident active processing in 2D-culture. Both cell lines formed spheres when grown in 3D suspension culture (Figure 2C). In MDA-MB-231 cells, robust increases in all forms were seen with 3D-culture (Figure 2D). Surprisingly, when Hs578T cells were grown in 3D-culture conditions, substantial levels of active form were now observed, as well as higher levels of ADAM8 precursor proform (Figure 2E). The ADAM8 proform in Hs578T spheres displayed a faster migration, similar to the band observed in MDA-MB-231 cells (Figure 5A), which may be due to altered posttranslational modifications. The precursor and active ADAM8 forms in Hs578T cells were better detected using LSBio and Millipore antibodies, respectively. Using two different specific *ADAM8* siRNAs led to effective knockdown of ADAM8 in these two lines under both growth conditions (Figure 2D and 2E). Altogether, ADAM8 was observed to be expressed and processed to an active form in TNBC cells, and increased levels were seen in cells grown in suspension as "tumorspheres".

### Example 19. TNBC Cell Migratory and Invasive Properties are Maintained by ADAM8

To test the role of ADAM8 on the aggressive phenotype of MDA-MB-231 and Hs578T cells *in vitro,* the effects of ADAM8 knockdown were measured on growth in soft agar, and migration and invasion through Matrigel, assays that are performed in 3D-culture conditions.

Decreased ADAM8 expression led to reduced abilities of both cell lines to grow under anchorage-independent conditions (Figure 3A), to migrate (Figure 3B) and invade through Matrigel (Figure 3C). Consistently, the capacity of these aggressive cells to form characteristic branching colonies in a Matrigel 3D-outgrowth assay was robustly inhibited following knockdown of ADAM8 expression (Figure 3D). The MDA-MB-231-derived cells, LM1 and LM2, which were selected *in vivo* for the cells' ability to metastasize to the lung (Minn et al. Nature 436:518-524, 2005), expressed higher levels of ADAM8 than the parental MDA-MB-231 line (Figure 3E), and more rapidly formed very large colonies in Matrigel (i.e., within 3-4 days vs 6-7 days for the parental cells; Figure 3F). The ability of LM1 and LM2 cells to form these highly invasive colonies in Matrigel was strikingly counteracted by ADAM8 knockdown (Figure 3E and 3F).

Thus, presence of ADAM8 was observed to be required for the maintenance of a migratory and invasive phenotype in TNBC cells in culture.

### Example 20. ADAM8 Promotes Tumor Growth, Spreading of Circulating Tumor Cells and Metastasis

The effects of stable ADAM8 knockdown *in vivo* was tested using a mouse orthotopic xenograft model. Clones of MDA-MB-231 cells expressing a specific *ADAM8* shRNA (shA8-17 and shA8-20) or *Control* shRNA (shCtrl-3 and shCtrl-5) were isolated. The *Control* shRNA clones expressed levels of ADAM8 comparable to the parental cells. Effective knockdown of ADAM8 expression by shADAM8 was verified by western blot (Figure 4A). The reduction in ADAM8 had no detectable effect on 2D-growth as assessed by an ATP assay (Figure 4B).

The two shADAM8 MDA-MB-231 clones showed significantly reduced ability both to migrate and to invade through Matrigel compared to the shControl clones (Figures 4C and 4D). ADAM8 (A8) or empty vector (EV) DNA was transiently transfected into shA8-20 MDA-MB-231 cells for 24 h. WCEs were isolated and subjected to Western blot analysis for ADAM8 (LSBio antibody) and tubulin (Figure 4E). Migration and Matrigel outgrowth assays were performed also as described in Figures 3B and 3D, respectively. Ectopic expression of ADAM8 in shA8-20 cells partially rescued the cells' ability to migrate (Figure 4F) and to form invasive colonies (Figure 4G), consistent with the observed transfection efficiency of about 40%. The rescue of these phenotypes by ectopic expression of ADAM8 in the shA8-20 clone demonstrated the role of ADAM8 knockdown in these cells.

To test the effects of ADAM8 knockdown on tumor growth and metastasis, 2.5 x 10⁶ MDA-MB-231 shCtrl-3 and shA8-20 cells were injected into the fourth inguinal mammary fat pad of female NOD/SCID mice (n = seven per group). Tumor growth at the orthotopic site was recorded by caliper measurements twice weekly. Between 13-16 days after cell implantation, mice from the shControl group started to develop mammary tumors that progressed with a high growth rate (Figure 4H). In marked contrast, tumors derived from the shA8-20 clone failed to grow beyond 0.05 cm³ even after more than 4 weeks (day 33). The time course was terminated when the tumors in the shControl group reached a size of about 1 cm³ as per the approved mouse protocol. The tumors were harvested, photographed and weighed (Figure 4I). A dramatic decrease in average tumor weight was measured (2.00 ± 0.08 vs 0.16 ± 0.01 g). Visually, the tumors derived from the shA8-20 clone appeared to be substantially less vascularized (Figure 4I).

CTCs were detected based on stable GFP expression in the MDA-MB-231 clones, using flow cytometry on freshly collected blood following termination of the time course. A profound decrease in the numbers of CTCs was observed with ADAM8 knockdown (12.8 ± 7.0 vs 0.0 ± 2.0 per µl of blood; Figure 4J). These data show that ADAM8 might be involved in the dissemination of CTCs; although the possibility that this relates to the large difference in tumor burden could not be excluded at that point. This question is addressed in further examples herein.

To test whether knockdown of ADAM8 affects metastasis, isolated brains were examined from six animals per group under a fluorescent dissecting microscope for the presence of GFP-positive metastases. All of the animals injected with shControl cells were observed to be positive for brain metastases (Figure 4K). In contrast, only one mouse with a very small metastasis was detected in the shADAM8 group (Figure 4K). Thus, knockdown of ADAM8 was observed to have profoundly impaired the capacity of MDA-MB-231 cell-derived mammary tumors to grow and spread in an orthotopic xenograft model, which is consistent with the high ADAM8 expression detected in distant metastases of breast cancer patients (Figures 1D and 1F).

### Example 21. Knockdown of ADAM8 Induces Angiogenic Tumor Dormancy

When solid tumors reach a few millimeters in diameter, nutrients and oxygen become insufficient and hypoxic stress is induced (Majmundar et al. Mol Cell 40:294-309, 2010). This leads to cell death by necrosis as well as to the release of factors that promote endothelial cell recruitment and angiogenesis that are needed to support continued tumor growth (Bergers et al. Nat Rev Cancer 3:401-410, 2003; Naumov et al. Cell Cycle 5:1779-1787, 2006). ADAM8 levels have been shown to be induced under hypoxia in pancreatic cancer cells (Valkovskaya et al. J Cel lMol Med 11:1162-1174, 2007). Thus, the effects of growth under reduced oxygen on ADAM8 expression in MDA-MB-231 and Hs578T breast cancer cells were measured.

Incubation of these cells under conditions of 1% of oxygen was observed to have led to substantial increases in ADAM8 protein levels (Figure 5A, left photograph). The levels of the ADAM8 proform and active form increased, and the proform now co-migrated with the band observed in MDA-MB-231 cells. Similar to the MDA-MB-231 parental cell line, ADAM8 expression was increased in shCtrl-3 cells under hypoxic conditions; whereas no induction of ADAM8 was seen under hypoxia in the shA8-20 clone (Figure 5A, right photograph). To test whether other breast cancer cell lines behave similarly to MDA-MB-231, Hs578T cells were cultured in adherent conditions (2D) or in suspension on ultra low-attachment plates (3D) for 48 h. MDA-MB-231 cells were cultured in adherent conditions only. Whole-cell extracts (WCEs) were analyzed by Western blotting for ADAM8 (LSBio antibody) and for β-Actin. (Figure 5B) As seen with MDA-MB-231 cells, ADAM8 proform is induced in Hs578T cells grown in 3D-cultures (Figure 5B).

To determine whether Hs578T cells behaved similarly under hypoxic conditions as compared to MDA-MB-231 cell lines, MDA-MB-231, Hs578T, shCtrl-3, and shA8-20 cells were incubated for 8 h under hypoxic conditions and nuclear extracts were subjected to Western blotting analysis for HIF-1α and Lamin B. All lanes were from the same gel, but two different times of exposure were used for the Hs578T cells (5 minutes) and MDA-MB-231-derived lines (30 minutes), as indicated by the vertical line. Therefore, HIF-1α is induced in various breast cancer cell lines under hypoxia (Figure 5C).

Consistently strong ADAM8 staining was detected around the necrotic areas in the shCtrl-3 tumors, and was absent in tumors derived from shA8-20 cells (Figure 5D), even though the extent of necrosis was similar in both tumor populations (Figure 5D).

To determine if expression levels of VEGF-A mRNA are altered by ADAM8 knockdown. RNA isolated from MDA-MB-231 shCtrl-3 or shA8-20 cells was assessed for VEGF-A expression by reverse transcription (RT)-PCR. Samples were normalized based on GAPDH expression levels. Results show that VEGF-A mRNA levels are unchanged in stable ADAM8 knockdown cells (Figure 5E).

To assess angiogenesis IHC staining of the tumors for the endothelial cell marker CD31 was performed. A significant decrease in the number of peritumoral vessels was measured in shA8-20 vs shCtrl-3 tumors (Figure 5F). Consistent with the model herein that tumors with higher ADAM8 levels are characterized by increased angiogenesis, a positive correlation was found between *ADAM8* and *CD31* (*PECAM1*) mRNA expression in tumor samples from patients with basal-like breast cancer (Figure 5G). Together, these findings indicate that in the absence of ADAM8, the MDA-MB-231-derived tumors underwent angiogenic dormancy and were thus unable to grow past a palpable size.

### Example 22. ADAM8 is Necessary for Release of Pro-Angiogenic Factors

To determine the mechanism whereby ADAM8 induces angiogenesis, the ability of conditioned media from MDA-MB-231 clones to stimulate the formation of vessel-like structures (tube formation) by human umbilical vein endothelial cells (HUVECs) on Matrigel was assessed (Figures 5H-5I). The shCtrl cells released factors that substantially increased the number of tubes formed by HUVECs compared to control media. In contrast, tube formation was severely reduced in the presence of conditioned medium from shA8 clones (Figures 5H-5I), indicating that ADAM8 promotes angiogenesis in part via a soluble factor.

To identify pro-angiogenic factors released by ADAM8, conditioned media from the two shControl and two shADAM8 MDA-MB-231 cell clones were analyzed using a Human Angiogenesis antibody array, which can detect the expression of 55 angiogenesis related proteins. Expression levels of the detected proteins were quantified and those that were significantly downregulated by more than an average of two-fold in the shADAM8 clones are presented in Figure 5J. Knockdown of ADAM8 led to substantial decreases in five important angiogenesis mediators (VEGF-A, angiogenin, platelet-derived growth factor PDGF-AA, Endothelin-1 and platelet growth factor P1GF), with the largest change seen in VEGF-A. To validate these results, expression of VEGF-A, a major inducer of angiogenesis and tube formation by endothelial cells (Lohela et al. Curr Opin Cell Biol 21:154-165, 2009; Mohammed et al. Br J Cancer 96:1092-1100, 2007), was examined using western blotting. A substantially decreased level of VEGF-A was seen in media of shA8 compared to shCtrl cells (Figure 5K). Quantification from this and two additional independent examples indicated that MDA-MB-231 cells released between 60-70% less VEGF-A (P = 0.002) into the media in cells in which ADAM8 is knocked down. Notably, release of VEGF-A from the shA8 cells could be restored by ectopic expression of full-length ADAM8 but not by the remnant form (Figure 5L), indicating that the metalloproteinase domain was required. Thus, in the absence of ADAM8, smaller amounts of VEGF-A and other pro-angiogenic factors are released, which can account for the reduction of angiogenesis observed *in vivo.*

To determine whether the remnant form of ADAM8 can induce VEGF-A, MDA-MB-231-derived shCtrl-3 or shA8-20 clones were transfected with the vectors expressing ADAM8 remnant form (Remnant) or with an empty vector (EV) DNA (Figure 5M). Cells were incubated with the indicated vector for 24 h. Then, the medium was replaced by serum-free medium and incubated for 16 h. Supernatants and cells were collected. Cells were lysed and WCEs were assessed by Western blotting for ADAM8 expression (Figure 5M). Data herein indicates that the remnant form of ADAM8 was unable to induce the release of VEGF-A from shA8-20 cells, in contrast to the full-length ADAM8.

To test ectopic expression of full-length ADAM8 and remnant form in stable ADAM8 knockdown cells, MDA-MB-231-derived shCtrl-3 or shA8-20 clones were transfected with the indicated vectors expressing ADAM8, its remnant form (Remnant) or empty vector (EV) DNA for 24 h. Then, the medium was replaced by serum-free medium and incubated for 16 h. Supernatants and cells were collected. Cells were lysed and WCEs were assessed by Western blotting for ADAM8 expression. VEGF-A released into the media was measured (Figure 5L). The data indicated that the remnant form was unable to induce the release of VEGF-A from shA8-20 cells, in contrast to the full-length ADAM8.

### Example 23. ADAM8 is Involved in Endothelial Cell Adhesion and Activation of β1-integrin

CTCs can be measured prior to formation of a detectable primary tumor (Eyles et al. J Clin Invest 120:2030-2039, 2010). Thus, the role of ADAM8 in the appearance of these early CTCs was investigated using a second set of mice (n = four/group; Figure 6A). Blood was collected at day seven, when tumors were barely detectable, and at days 14 and 21, when a difference in size was initially observed and measured. CTCs were detected as early as seven days after cell implantation. The numbers of CTCs were significantly lower in the mice injected with shA8-20 vs shCtrl-3 cells throughout the time course (Figure 6A). This suggests a lower potential risk for developing metastases to distant organs when ADAM8 is knocked down, which is independent of the tumor size.

Binding of primary tumor cells and then CTCs to an endothelial layer followed by transmigration through the blood vessel wall (intravasation and extravasation, respectively) are critical steps in metastasis (Scott et al. Nat Rev Cancer 12:445-446, 2012; Steeg Nat Med 12:895-904, 2006). Thus, the effects of ADAM8 on binding of MDA-MB-231 cells to HUVECs were determined. shControl and shADAM8 cells were added to tissue culture plates in the presence or absence of a HUVEC monolayer. The two shControl clones were observed to adhere much more efficiently to a confluent HUVEC monolayer than the two shADAM8 clones, and adhesion on plastic was not affected by ADAM8 expression (Figure 6B). Furthermore, in a transendothelial cell migration assay, shADAM8 cells were much less effective in migrating through a confluent HUVEC monolayer than the shControl cells (Figure 6C). Thus, ADAM8 promotes tumor cell adhesion onto and migration through an endothelial cell layer, as found in a vessel wall.

The MDA-MB-231-derived shCtrl-3 clone and the total MDA-MB-231 population were transfected with either Control siRNA (siCtrl) or two specific ADAM8 siRNAs (siA8) for 72 h. Cancer cell adhesion to (Figure 6D) and transendothelial migration through (Figure 6E) HUVECs were assessed as in Figure 6B and 6C, respectively. *P < 0.05, Student's t test. Results show that siRNA-mediated knockdown of ADAM8 in MDA-MB-231 cells reduces ability to adhere to and transmigrate through HUVECs.

WCEs from HUVECs and MDA-MB-231 cells, as a positive control for ADAM8 expression, were subjected to western blotting for ADAM8 (LSBio antibody) and tubulin (Figure 6F). ADAM8 was not detected in HUVECs which indicates that ADAM8 participates in the establishment of cell-cell contact between breast tumor cells and endothelial cells. HUVECs being observed to not express ADAM8 under normal conditions of culture excludes the possibility that ADAM8 forms homodimers in *trans,* i.e., between tumoral and endothelial cells. Betal-integrin has been shown to be involved in adhesion of MDA-MB-231 cells onto an endothelial cell monolayer (Price et al. Br J Cancer 74:1762-1766, 1996).

Activation of β1-integrin in shCtrl-3 cells was observed to be required for adhesion to HUVECs, as judged by reduced endothelial cell-binding *in vitro* following addition of an antagonist β1-integrin antibody (Figure 6G). A monoclonal antibody directed against the metalloproteinase and disintegrin domains of ADAM8 (Mab1031) was observed to have inhibited the adhesion of shCtrl-3 cells to HUVECs, essentially to the same extent as the β1-integrin antagonist antibody (Figure 6H) and also to have prevented ability of these cells to transmigrate through an endothelial cell monolayer (Figure 6I). Further, another monoclonal ectodomain antibody (Mab10311) that targets the extracellular cysteine-rich and EGF-like domains of ADAM8, had similar effects (Figures 6H and 6I). Importantly, using an antibody specific for the activated form of β1-integrin (12G10), strong β1-integrin activation was observed in the mammary tumors derived from shCtrl-3 cells, notably at the peritumoral area, but was absent in shA8-20 tumors and in the contralateral mammary glands of shCtrl mice (Figure 6J), consistent with the model proposed herein that ADAM8 promotes tumor cell adhesion onto the endothelium and dissemination by β1-integrin activation *in vivo.*

### Example 24. ADAM8 MP/DI Ectodomain Antibody Reduces Tumor Growth and Dissemination

To validate ADAM8 as a therapeutic target for TNBC, the Mab1031 ADAM8 antibody was selected for use in an orthotopic model.

Mice were treated with either 0.5 mg/kg of Mab1031 antibody (n = 9) or a control isotype-matched IgG2B (n = 8) by i.p. injection twice weekly from the day of implantation of shCtrl-3 cells. Anti-ADAM8 treatment was observed to have resulted in significantly decreased primary tumor burden by about 50% (Figure 7A) and tumor weight by about 36% (Figure 7B) after three weeks of treatment. In animals treated with anti-ADAM8, a substantial reduction was seen also in the size and numbers of brain metastases per mouse, and in the frequency of brain metastases (Figure 7C). Angiogenesis surrounding the tumors (Figure 7D) and VEGF-A levels in the tumors (Figure 7E) were similarly reduced by treatment with the ADAM8 antibody. The ADAM8 metalloproteinase substrate CD23 (Koller et al. Curr Pharm Des 15:2272-2281, 2009) was used in an *in vitro* analysis.

Anti-ADAM8 MP/DI antibody inhibits the metalloproteinase activity of ADAM8. HEK293 cells were co-transfected with vectors expressing CD23, which is a well-established substrate of ADAM8 protease activity, and either ADAM8 or its remnant form or EV DNA. Six hours later, the medium was replaced with serum-free medium. After 16 h, supernatants were collected and volumes corresponding to equal cell numbers were assessed for cleaved CD23 (29 kDa) using Western blotting. Cells were harvested and WCEs were assessed for expression of ADAM8 and tubulin. All lanes were from the same gel, but cut to re-align as indicated by the vertical line (Figure 7F, left). In the presence of full-length ADAM8 but not its remnant form lacking the metalloproteinase domain, CD23 is cleaved with release of a diagnostic soluble 29 kDa fragment (Figure 7F, right). HEK293 cells were co-transfected with vectors expressing CD23 and ADAM8. Six hours later, medium was replaced with serum-free medium in the presence of 10 µg/ml of either anti-ADAM8 antibody (Mab1031) or isotype control IgG2B (Mab004). After 16 h, we measured cleaved 29 kDa CD23 in supernatants and ADAM8 expression in WCEs, as described above. In the presence of the anti-ADAM8 MP/DI antibody, the cleavage of CD23 by ADAM8 was substantially reduced. (Figure 7F, right) In the presence of an ADAM8 antibody, the *in vitro* analysis showed a substantial reduction in activity indicating inhibition of metalloproteinase by the antibody (Figure 7F).

### Example 25. ADAM8 MP/DI Ectodomain Antibody Reduces Dissemination of Pre-Existing Tumors in a Neo-Adjuvant Setting

To test the use of this ADAM8 antibody in a more clinically relevant setting, a tumor resection model was used (Figure 7G). Ability to treat pre-existing tumors with ADAM8 antibody was tested.

Twelve days after MDA-MB-231 shCtrl-3 cells were implanted into the MFP of mice, palpable tumors were observed, and treatment was initiated with 1.5 mg/kg of anti-ADAM8 MP/DI antibody (Mab1031) or isotype control IgG2B (Mab004). Additional doses of antibody were further administered on day 15 and on day 17, at which point tumors reached a volume of about 0.15 cm³ and tumor resection was performed. Antibody treatment was continued twice weekly for a 5-week period, and then organ metastasis was examined using fluorescent microscopy (Figure 7H).

In the isotype-treated control group, metastases to the brain and lungs were seen in 8/8 and 7/8 mice, respectively. In the anti-ADAM8-treated group only 2/9 and 1/9 mice showed metastases in the brain and lungs. Thus, treatment with an anti-ADAM8 antibody resulted in a profound reduction in the frequency of metastases to both the brain and lungs compared to the frequency of metastases in the isotype control antibody treated group.

### Example 26. Knockdown of ADAM8 Reduces Ability of TNBC to Colonize Distant Organs

To test the role of ADAM8 in the ability of breast cancer cells to colonize typical distant metastasis sites, MDA-MB-231-derived shCtrl-3 and shA8-20 cells, which stably express Luciferase, were injected in the left ventricle of female NOD/SCID mice (n = 4/group). Total tumor burden was monitored weekly by Luciferase activity measurement using the Xenogen Biophotonic Imaging system. Organ and bone metastases were assessed using this method at sacrifice.

Cardiac injection of MDA-MB-231-derived shA8-20 cells caused a significantly lower total tumor burden after 2 or 3 weeks compared to injection of shCtrl-3 cells (Figures 8A and 8B). At the termination of the example, shCtrl-3 cells were consistently found to colonize numerous organs and bones, in contrast to the control shA8-20 cells (Figure 8C). Thus, ADAM8 was observed to be required for colonization of distant metastasis sites by TNBC cells.

### Example 27. ADAM8 CRD/ELD Ectodomain Antibody Reduces Tumor Dissemination

Since treatment of breast cancer cells with Mab10311 (CRD/ELD) and Mab1031 (MP/DI) antibodies reduced amount of transendothelial cell migration through a HUVEC monolayer to about the same extent (Figures 6I and 12A), targeting of the CRD/ELD region of ADAM8 was tested for inhibition of tumor growth and dissemination of TNBC in an orthotopic model. The Mab10311 (CRD/ELD) antibody and the Mab1031 (MP/DI) antibody ability to inhibit the MP activity of ADAM8 were compared. The Mab10311 was observed to inhibit MP activity about 40%, which was a lesser extent than Mab1031, 70% (Figure 12B). The Mab10311 ADAM8 antibody was then tested in the orthotopic model. Mice were treated with 1.5 mg/kg of either Mab10311 antibody (n = 7) or a control isotype-matched IgG1 (n = 7) by i.p. injection twice weekly from the day of implantation of shCtrl-3 cells.

After three weeks, no significant change in primary tumor burden was seen following the anti-ADAM8 Mab10311 treatment (Figure 12C, left bar graph). However, a striking reduction in the size and number of brain metastases per mouse and in the overall frequency of metastases was observed in animals treated with anti-ADAM8 Mab10311 antibody (Figure 12C, right chart). These results indicate that inhibition of the DI activity of ADAM8 by interaction of an antibody with CRD/ELD regions was sufficient to profoundly reduce TNBC tumor dissemination. These data further indicate that inhibition of the MP activity, which is required for release of pro-angiogenic factors, is necessary to prevent tumor growth. Thus, the data herein indicate that inhibition of activities of the MP and DI domains of ADAM8 are required to prevent both tumor growth and dissemination.

Overall, data herein validate ADAM8 as a therapeutic target for TNBC. ADAM8, which is abundantly expressed in aggressive patient breast tumors and metastases, was shown to be essential for tumor growth and dissemination in orthotopic mouse models using knockdown and antibody strategies. Notably, treatment of pre-existing tumors with an antibody targeting the MP/DI domains of ADAM8 profoundly reduced tumor ability to metastasize in a resection model. Two essential novel roles for ADAM8 in tumor progression were identified (Figure 9). ADAM8 facilitates the release of pro-angiogenic factors, including VEGF-A, into the tumor microenvironment, leading to neovascularization and continued tumor growth. ADAM8 also activates β1-integrin on the tumor cells permitting attachment of the cells to the vascular endothelium and entry into the blood circulation. Increased numbers of CTCs raise the risk of developing metastases in distant organs such as brain and lungs. TNBCs are highly aggressive and more likely to spread compared to other breast tumors, and no targeted treatment options are currently available. Given the importance of ADAM8 in growth and dissemination of TNBC, and its advantageous localization on the tumor cell surface and the lack of a phenotype of ADAM8 knockout mice. Data herein identify ADAM8 as a promising and novel druggable target for the treatment of breast cancers.

Binding of integrins to cell-surface adhesion molecules and extracellular matrix proteins governs cell adhesion and shape via transduction of intracellular signals (Avraamides et al. Nat Rev Cancer 8:604-617, 2008; Desgrosellier et al. Nat Rev Cancer 10:9-22, 2010). Betal-integrin was found to mediate adhesion of breast cancer and other tumor cells to quiescent endothelial cells (Price et al. Br J Cancer 74:1762-1766, 1996). Several ADAM proteins, which contain RGD-like integrin-binding motifs in the disintegrin domain, constitute ligands for integrins (Bridges & Bowditch, Curr Pharm Des 11:837-847, 2005). Notably, an antibody targeting of the MP/DI domains of ADAM8 was observed to have greatly reduced the ability of TNBC cells to bind endothelial cells *in vitro* and to disseminate in mice. In addition, another antibody directed against the ADAM8 CRD/ELD domains was equally effective *in vitro,* suggesting allosteric hindrance or altered ADAM8 structure may also play a role. This antibody consistently inhibited dissemination *in vivo.*

Early in the tumorigenesis process, solid tumors stop increasing in mass (tumor dormancy) unless they can overcome hypoxic stress by stimulating angiogenesis, which provides oxygen and nutrients essential for further tumor growth (Bergers et al. Nat Rev Cancer 3:401-410, 2003; Majmundar et al. Mol Cell 40:294-309, 2010; Naumov et al. Cell Cycle 5:1779-1787, 2006). In TNBC cells, hypoxia was observed to have induced expression of ADAM8 proform and active processed form. Mammary tumors of shCtrl cells displayed a substantial increase in ADAM8 protein around necrotic areas, but not in tumors of shA8 animals. Using Transfac analysis, several putative Hypoxia Response Elements were identified in the *ADAM8* promoter. The extent of HIF-1α induction and amount of necrosis in tumors derived in shCtrl compared to shA8 cells was similar (Figure 5C), and ADAM8 knockdown does not alter the initial response to hypoxia. High ADAM8 levels were also seen in patient TNBCs, especially in the metastases to the brain, consistent with induction related to hypoxic stress. Altogether, these findings are consistent with the model envisioned herein that induction of ADAM8 is required for breast tumors to overcome hypoxic stress.

Activation of the VEGFR2 tyrosine kinase receptor by VEGF-A, which promotes endothelial cell proliferation, sprouting, migration, as well as vascular permeability, is a major pathway inducing angiogenesis (Lohela et al. Curr Opin Cell Biol 21:154-165, 2009). High VEGF-A expression has been associated with increased microvessel density, metastasis, and a shorter overall survival in patients with primary invasive breast cancer (Mohammed et al. Br J Cancer 96:1092-1100, 2007). VEGF-A activity is regulated to a large extent in the extracellular space, where cell-surface or extracellular matrix bound VEGF-A reservoirs can be made bioavailable through proteolytic cleavage by various enzymes, including metalloproteinases MMP-3, -7 and -9. (Hawinkels et al. Eur J Cancer 44:1904-1913, 2008; Lee et al. J Invest Dermatol 130:763-773, 2005). TNBCs with higher intratumoral VEGF-A levels compared to non-TNBCs have a shorter time to relapse (Linderholm et al. Ann Oncol 20:1639-1646, 2009).

Knockdown of ADAM8 in MDA-MB-231 cells resulted in reduced VEGF-A levels in conditioned media, but did not appear to affect *VEGF-A* mRNA levels (Figure 5E). Consistently, both ADAM8 antibody treatment and knockdown were observed herein to have reduced tumor angiogenesis. As a positive correlation was identified between *ADAM8* and *CD31* mRNA levels in basal-like tumors, high ADAM8 levels appear associated with increased tumor vascularization in these patients. Data herein show that the metalloproteinase activity of ADAM8 plays an essential role in processing or release of VEGF-A and potentially other pro-angiogenic factors.

Biological roles of ADAM proteins in malignancy identified in Duffy et al. (Duffy et al. Clin Proteomics, 8:9, 2011; Maretzky et al. Nat Commun 2:229, 2011; Duffy et al. Clin Cancer Res 15:1140-1144, 2009). ADAM15 cytoplasmic domain variants have been implicated in mammary carcinoma (Zhong et al. Mol Cancer Res 6:383-394, 2008). An ADAM12 secreted isoform promotes breast tumor metastasis *in vivo* (Roy et al. J Biol Chem 286:20758-20768, 2011). ADAM10 is upregulated in melanoma metastasis compared to primary melanoma (Lee et al. J Invest Dermatol 130:763-773, 2010). ADAM8 expression was previously correlated with tumor stage or an unfavorable prognosis for patients with esophageal-gastric, lung, pancreatic or brain cancers, and with invasiveness of tumor cells in culture (Baren et al. Br J Cancer 107:143-149, 2012; Koller et al. Curr Pharm Des 15:2272-2281, 2009), although little was explored about its mechanism of action. No evidence of *ADAM8* gene amplification in these cancers was reported. Several pathways commonly dysregulated in aggressive malignancies were found to induce *ADAM8* mRNA levels, e.g., RelB NF-κB and EGFR (Sriraman et al. Biol Reprod 78:1038-1048, 2008).

In examples herein, ADAM8 expression levels were correlated with a poor prognosis in breast cancer patients and concomitantly with increased numbers of CTCs and metastases in an orthotopic mouse model. CTCs and micrometastases have been recently shown to occur early during the progression of cancers from various origins (melanoma, breast and cervical cancers; Toh et al. Immunol Res 53:229-234, 2012). ADAM8 and ADAM12 activities measured in urine samples to identify breast cancer patients with invasive and metastatic disease (Roy et al. Clin Biochem 44:1434-1439, 2011).

Data herein show that the potential use of monitoring ADAM8 in a non-invasive method as a diagnostic/prognostic biomarker for early cancer detection or potentially to follow disease progression and recurrence, and to treat breast cancer and prevent metastasis is feasible.

## Claims

1. An ADAM8 ectodomain-binding antibody or the antigen-binding fragment thereof for use in the treatment of breast cancer or breast cancer associated metastasis **characterized in that** the antibody or the antigen-binding fragment thereof binds to SEQ ID No 9 and that the antibody or the antigen-binding fragment thereof inhibits the activity of the metalloproteinase domain and disintegrin domain of ADAM8.

2. The ADAM8 antibody or the antigen-binding fragment thereof for use according to claim 1 wherein the breast cancer is ductal carcinoma *in situ* or triple negative.

3. A pharmaceutical composition for use in the treatment of breast cancer or breast cancer associated metastasis comprising:
a therapeutically effective amount of the antibody according to any of the previous claims or the antigen-binding fragment thereof and a pharmaceutically acceptable carrier.

4. The pharmaceutical composition for use according to claim 3, wherein the composition comprises a plurality of antibodies or antigen-binding fragments thereof inhibiting the activity of the metalloproteinase domain and disintegrin domain of ADAM8.

## Patentansprüche

1. ADAM8-Ektodomäne bindender Antikörper oder das Antigenbindungsfragment desselben zur Verwendung bei der Behandlung von Brustkrebs oder brustkrebsassoziierter Metastasierung, **dadurch gekennzeichnet, dass** der Antikörper oder das Antigenbindungsfragment desselben an SEQ ID NO:9 bindet und dass der Antikörper oder das Antigenbindungsfragment desselben die Aktivität der Metallproteinasedomäne und Disintegrindomäne von ADAM8 hemmt.

2. ADAM8-Antikörper oder das Antigenbindungsfragment desselben zur Verwendung gemäß Anspruch 1, wobei der Brustkrebs duktales Karzinom *in situ* oder dreifach negativ ist.

3. Pharmazeutische Zusammensetzung zur Verwendung bei der Behandlung von Brustkrebs oder brustkrebsassoziierter Metastasierung, umfassend:
eine therapeutisch wirksame Menge des Antikörpers gemäß einem der vorhergehenden Ansprüche oder des Antigenbindungsfragments desselben und einen pharmazeutisch akzeptablen Träger.

4. Pharmazeutische Zusammensetzung zur Verwendung gemäß Anspruch 3, wobei die Zusammensetzung mehrere Antikörper oder Antigenbindungsfragmente derselben umfasst, welche die Aktivität der Metallproteinasedomäne und Disintegrindomäne von ADAM8 hemmen.

## Revendications

1. Anticorps de liaison d'ectodomaine ADAM8 ou fragment de liaison d'antigène de celui-ci pour une utilisation dans le traitement du cancer du sein ou de métastases associées au cancer du sein, **caractérisé en ce que** l'anticorps ou le fragment de liaison d'antigène de celui-ci se lie à l'ID de SEQ n° 9 et **en ce que** l'anticorps ou le fragment de liaison d'antigène de celui-ci inhibe l'activité du domaine de métalloprotéinase et du domaine de désintégrine ADAM8.

2. Anticorps ADAM8 ou fragment de liaison d'antigène de celui-ci en vue d'une utilisation selon la revendication 1, dans lequel le cancer du sein est un carcinome canalaire *in situ* ou triple négatif.

3. Composition pharmaceutique en vue de l'utilisation dans le traitement du cancer du sein ou d'une métastase associée au cancer du sein, comprenant :
un volume efficace thérapeutiquement de l'anticorps selon une quelconque des revendications précédentes ou de son fragment de liaison d'antigène et un support pharmaceutiquement acceptable.

4. Composition pharmaceutique en vue de l'utilisation selon la revendication 3, dans laquelle la composition comprend une pluralité d'anticorps ou de fragments de liaison d'antigène de ceux-ci inhibant l'activité du domaine de métalloprotéinase et du domaine de désintégrine ADAM8.


### SEQUENCE LISTING

<110> Tufts University
<120> Methods and compositions for prognosis, diagnosis and treatment of ADAM8-expressing cancer
<130> 34724-129
<140> PCT/US 14/
   <141> 2014-05-13
<150> 61/822,738
   <151> 2013-05-13
<150> 61/898,830
   <151> 2013-11-01
<160> 9
<170> PatentIn version 3.5
<210> 1
   <211> 21
   <212> RNA
   <213> Artificial Sequence
<220>
   <223> The sequence was designed and synthesized
<400> 1
   cggcaccugc augacaacgu a 21
<210> 2
   <211> 21
   <212> RNA
   <213> Artificial Sequence
<220>
   <223> The sequence was designed and sythesized
<400> 2
   cugcgcgaag cugcugacug a 21
<210> 3
   <211> 824
   <212> PRT
   <213> Artificial Sequence
<220>
   <223> The sequence was designed and sythesized
<400> 3
<210> 4
   <211> 30
   <212> PRT
   <213> Oryctolagus cuniculus
<400> 4
   Pro Gly Pro Ala Glu Gly Ala Val Gly Pro Lys Val Ala Leu Lys Pro
   1 5 10 15
   Pro Ile Gln Arg Lys Gln Gly Ala Gly Ala Pro Thr Ala Pro
   20 25 30
<210> 5
   <211> 61
   <212> PRT
   <213> Oryctolagus cuniculus
<400> 5
   Pro Val Tyr Thr Arg Gln Ala Pro Lys Gln Val Ile Lys Pro Thr Phe
   1 5 10 15
   Ala Pro Pro Val Pro Pro Val Lys Pro Gly Ala Gly Ala Ala Asn Pro
   20 25 30
   Gly Pro Ala Glu Gly Ala Val Gly Pro Lys Val Ala Leu Lys Pro Pro
   35 40 45
   Ile Gln Arg Lys Gln Gly Ala Gly Ala Pro Thr Ala Pro
   50 55 60
<210> 6
   <211> 155
   <212> PRT
   <213> Mus musculus
<400> 6
   Ser Gly Gly Tyr Cys Tyr Asn Gly Ala Cys Pro Thr Leu Ala Gln Gln
   1               5                   10                  15
   Cys Gln Ala Phe Trp Gly Pro Gly Gly Gln Ala Ala Glu Glu Ser Cys
               20                  25                  30
   Phe Ser Tyr Asp Ile Leu Pro Gly Cys Lys Ala Ser Arg Tyr Arg Ala
           35                  40                  45
   Asp Met Cys Gly Val Leu Gln Cys Lys Gly Gly Gln Gln Pro Leu Gly
       50                  55                  60
   Arg Ala Ile Cys Ile Val Asp Val Cys His Ala Leu Thr Thr Glu Asp
   65                  70                  75                  80
   Gly Thr Ala Tyr Glu Pro Val Pro Glu Gly Thr Arg Cys Gly Pro Glu
                   85                  90                  95
   Lys Val Cys Trp Lys Gly Arg Cys Gln Asp Leu His Val Tyr Arg Ser
               100                 105                 110
   Ser Asn Cys Ser Ala Gln Cys His Asn His Gly Val Cys Asn His Lys
           115                 120                 125
   Gln Glu Cys His Cys His Ala Gly Trp Ala Pro Pro His Cys Ala Lys
       130                 135                 140
   Leu Leu Thr Glu Val His Ala Ala Ser Gly Ser
   145                 150                 155
<210> 7
   <211> 495
   <212> PRT
   <213> Capra hicus
<400> 7
   His Leu Leu Gln Thr Ala Gly Thr Cys Gly Val Ser Asp Asp Ser Leu
   1               5                   10                  15
   Gly Ser Leu Leu Gly Pro Arg Thr Ala Ala Val Phe Arg Pro Arg Pro
               20                  25                  30
   Gly Asp Ser Leu Pro Ser Arg Glu Thr Arg Tyr Val Glu Leu Tyr Val
           35                  40                  45
   Val Val Asp Asn Ala Glu Phe Gln Met Leu Gly Ser Glu Ala Ala Val
       50                  55                  60
   Arg His Arg Val Leu Glu Val Val Asn His Val Asp Lys Leu Tyr Gln
   65                  70                  75                  80
   Lys Leu Asn Phe Arg Val Val Leu Val Gly Leu Glu Ile Trp Asn Ser
                   85                  90                  95
   Gln Asp Arg Phe His Val Ser Pro Asp Pro Ser Val Thr Leu Glu Asn
               100                 105                 110
   Leu Leu Thr Trp Gln Ala Arg Gln Arg Thr Arg Arg His Leu His Asp
           115                 120                 125
   Asn Val Gln Leu Ile Thr Gly Val Asp Phe Thr Gly Thr Thr Val Gly
       130                 135                 140
   Phe Ala Arg Val Ser Ala Met Cys Ser His Ser Ser Gly Ala Val Asn
   145                 150                 155                 160
   Gln Asp His Ser Lys Asn Pro Val Gly Val Ala Cys Thr Met Ala His
                   165                 170                 175
   Glu Met Gly His Asn Leu Gly Met Asp His Asp Glu Asn Val Gln Gly
               180                 185                 190
   Cys Arg Cys Gln Glu Arg Phe Glu Ala Gly Arg Cys Ile Met Ala Gly
           195                 200                 205
   Ser Ile Gly Ser Ser Phe Pro Arg Met Phe Ser Asp Cys Ser Gln Ala
       210                 215                 220
   Tyr Leu Glu Ser Phe Leu Glu Arg Pro Gln Ser Val Cys Leu Ala Asn
   225                 230                 235                 240
   Ala Pro Asp Leu Ser His Leu Val Gly Gly Pro Val Cys Gly Asn Leu
                   245                 250                 255
   Phe Val Glu Arg Gly Glu Gln Cys Asp Cys Gly Pro Pro Glu Asp Cys
               260                 265                 270
   Arg Asn Arg Cys Cys Asn Ser Thr Thr Cys Gln Leu Ala Glu Gly Ala
           275                 280                 285
   Gln Cys Ala His Gly Thr Cys Cys Gln Glu Cys Lys Val Lys Pro Ala
       290                 295                 300
   Gly Glu Leu Cys Arg Pro Lys Lys Asp Met Cys Asp Leu Glu Glu Phe
   305                 310                 315                 320
   Cys Asp Gly Arg His Pro Glu Cys Pro Glu Asp Ala Phe Gln Glu Asn
                   325                 330                 335
   Gly Thr Pro Cys Ser Gly Gly Tyr Cys Tyr Asn Gly Ala Cys Pro Thr
   340 345 350
   Leu Ala Gln Gln Cys Gln Ala Phe Trp Gly Pro Gly Gly Gln Ala Ala
   355 360 365
   Glu Glu Ser Cys Phe Ser Tyr Asp Ile Leu Pro Gly Cys Lys Ala Ser
   370 375 380
   Arg Tyr Arg Ala Asp Met Cys Gly Val Leu Gln Cys Lys Gly Gly Gln
   385 390 395 400
   Gln Pro Leu Gly Arg Ala Ile Cys Ile Val Asp Val Cys His Ala Leu
   405 410 415
   Thr Thr Glu Asp Gly Thr Ala Tyr Glu Pro Val Pro Glu Gly Thr Arg
   420 425 430
   Cys Gly Pro Glu Lys Val Cys Trp Lys Gly Arg Cys Gln Asp Leu His
   435 440 445
   Val Tyr Arg Ser Ser Asn Cys Ser Ala Gln Cys His Asn His Gly Val
   450 455 460
   Cys Asn His Lys Gln Glu Cys His Cys His Ala Gly Trp Ala Pro Pro
   465 470 475 480
   His Cys Ala Lys Leu Leu Thr Glu Val His Ala Ala Ser Gly Ser
   485 490 495
<210> 8
   <211> 29
   <212> RNA
   <213> Artificial Sequence
<220>
   <223> The sequence was designed and synthesized
<400> 8
   gcggcaccug caugacaacg uacagcuca 29
<210> 9
   <211> 298
   <212> PRT
   <213> Mus musculus
<400> 9
   Tyr Val Glu Leu Tyr Val Val Val Asp Asn Ala Glu Phe Gln Met Leu
   1               5                   10                  15
   Gly Ser Glu Ala Ala Val Arg His Arg Val Leu Glu Val Val Asn His
   20 25 30
   Val Asp Lys Leu Tyr Gln Lys Leu Asn Phe Arg Val Val Leu Val Gly
   35 40 45
   Leu Glu Ile Trp Asn Ser Gln Asp Arg Phe His Val Ser Pro Asp Pro
   50 55 60
   Ser Val Thr Leu Glu Asn Leu Leu Thr Trp Gln Ala Arg Gln Arg Thr
   65 70 75 80
   Arg Arg His Leu His Asp Asn Val Gln Leu Ile Thr Gly Val Asp Phe
   85 90 95
   Thr Gly Thr Thr Val Gly Phe Ala Arg Val Ser Ala Met Cys Ser His
   100 105 110
   Ser Ser Gly Ala Val Asn Gln Asp His Ser Lys Asn Pro Val Gly Val
   115 120 125
   Ala Cys Thr Met Ala His Glu Met Gly His Asn Leu Gly Met Asp His
   130 135 140
   Asp Glu Asn Val Gln Gly Cys Arg Cys Gln Glu Arg Phe Glu Ala Gly
   145 150 155 160
   Arg Cys Ile Met Ala Gly Ser Ile Gly Ser Ser Phe Pro Arg Met Phe
   165 170 175
   Ser Asp Cys Ser Gln Ala Tyr Leu Glu Ser Phe Leu Glu Arg Pro Gln
   180 185 190
   Ser Val Cys Leu Ala Asn Ala Pro Asp Leu Ser His Leu Val Gly Gly
   195 200 205
   Pro Val Cys Gly Asn Leu Phe Val Glu Arg Gly Glu Gln Cys Asp Cys
   210 215 220
   Gly Pro Pro Glu Asp Cys Arg Asn Arg Cys Cys Asn Ser Thr Thr Cys
   225 230 235 240
   Gln Leu Ala Glu Gly Ala Gln Cys Ala His Gly Thr Cys Cys Gln Glu
   245 250 255
   Cys Lys Val Lys Pro Ala Gly Glu Leu Cys Arg Pro Lys Lys Asp Met
   260 265 270
   Cys Asp Leu Glu Glu Phe Cys Asp Gly Arg His Pro Glu Cys Pro Glu
           275                 280                 285
   Asp Ala Phe Gln Glu Asn Gly Thr Pro Cys
       290                 295